# EUROPEAN PATENT SPECIFICATION

(11) **EP 4 215 537 B1**
(45) Date of publication and mention of the grant of the patent: **29.04.2026**
(21) Application number: 21869730.8
(22) Date of filing: 15.09.2021
(51) Int. Cl.: C07F 9/46, C07C 2/36, C07C 11/02, C07C 11/107, B01J 31/18

(54) **LIGAND COMPOUND, ORGANOCHROMIUM COMPOUND, AND CATALYST SYSTEM COMPRISING THE SAME**
LIGANDENVERBINDUNG, ORGANOCHROMVERBINDUNG UND KATALYSATORSYSTEM DAMIT
COMPOSÉ LIGAND, COMPOSÉ ORGANOCHROME ET SYSTÈME CATALYTIQUE LES COMPRENANT

(30) Priority: 15.09.2020 KR 20200118561; 31.05.2021 KR 20210070100
(43) Date of publication of application: 26.07.2023
(73) Proprietor: LG Chem, Ltd., Seoul 07336 (KR)
(72) Inventor: KIM, Seok Sun, Daejeon 34122 (KR); KIM, Tae Hee, Daejeon 34122 (KR); SA, Seok Pil, Daejeon 34122 (KR); KIM, Se Young, Daejeon 34122 (KR); SHIN, Eun Ji, Daejeon 34122 (KR)
(74) Representative: Goddar, Heinz J.
(86) International application number: PCT/KR2021/012636
(87) International publication number: WO 2022/060108

(56) References cited:
- WO-A1-2017/205149
- CA-C- 2 703 435
- CN-A- 105 754 019
- CN-A- 105 754 019
- KR-A- 20130 142 151
- KR-A- 20150 006 474
- KR-A- 20190 029 663

## Description

### TECHNICAL FIELD

### [Cross-reference to Related Applications]

The present application claims the benefit of priority based on Korean Patent Application Nos. 10-2020-0118561, filed on September 15, 2020, 10-2021-0070100, filed on May 31, 2021, and 10-2021-0123295, 10-2021-0123296 and 10-2021-0123303, filed on September 15, 2021.

### [Technical Field]

The present invention relates to a ligand compound, an organochromium compound, and a catalyst system comprising the same.

### BACKGROUND ART

A linear alpha-olefin like 1-hexene and 1-octene is used as a detergent, a lubricant, a plasticizer, or the like, and particularly, used as a comonomer for adjusting the density of a polymer during preparing a linear low-density polyethylene.

In the preparation process of the conventional linear low-density polyethylene (LLDPE), in order to adjust the density by forming a branch on a polymer backbone, copolymerization with a comonomer like an alpha-olefin such as 1-hexene and 1-octene together with ethylene has been performed.

Accordingly, in order to prepare the LLDPE with the high comonomer content, there were defects of the cost of the comonomer accounted for a big part of the manufacturing cost, and to solve such defects, various attempts have been conducted.

Particularly, the LLDPE is mainly produced through a shell higher olefin process. However, according to the method, alpha-olefins with various lengths are synthesized simultaneously according to Schultz-Flory distribution, and a separate separation process is inconveniently required for obtaining a specific alpha-olefin.

To solve such defects, a method of selectively synthesizing 1-hexene through the trimerization reaction of ethylene, or selectively synthesizing 1-octene through the tetramerization reaction of ethylene has been suggested. In addition, more studies on a catalyst system capable of selectively oligomerizing ethylene have been conducted.

### [Prior Art Document]

### [Patent Document]

(Patent Document 1) Korean Laid-open Patent No. 10-2014-0124732

CN 105 754 019 A a catalyst composition for preparing long-chain branch wide/bimodal polyethylene by virtue of insitu copolymerization. The catalyst composition comprises an oligomerization catalyst, a first cocatalyst, a polymerization catalyst and a second cocatalyst.

CA 2 703 435 C discloses oligomerization of ethylene using a chromium catalyst having a phosphorus-nitrogen-phosphorus ("P-N-P") ligand is typically activated with an aluminoxane.

### DISCLOSURE OF THE INVENTION

### TECHNICAL PROBLEM

An object of the present invention is to provide an organochromium compound of a novel structure, which shows high catalyst activity and selectivity and may produce a linear alpha-olefin with excellent efficiency, and a catalyst system including the same.

### TECHNICAL SOLUTION

In order to solve the above-described task, the present invention provides a ligand compound represented by Formula 1 below.

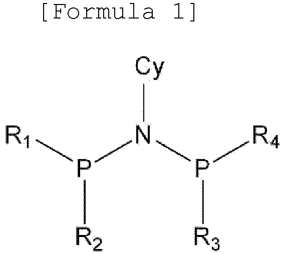

In Formula 1,
Cy is cycloalkyl of 5 to 10 carbon atoms, or cycloalkyl of 5 to 10 carbon atoms, fused with aryl of 6 to 10 carbon atoms, and
R₁ to R₄ are each independently aryl of 6 to 20 carbon atoms, which is para-substituted with a substituent selected from the group consisting of cycloalkyl of 5 to 20 carbon atoms, alkyl of 6 to 20 carbon atoms, alkoxyalkyl of 6 to 20 carbon atoms, arylalkoxyalkyl of 10 to 30 carbon atoms and trialkylsilyl of 3 to 30 carbon atoms.

In addition, the present invention provides an organochromium compound including the ligand compound represented by Formula 1; and chromium (Cr) coordinated with the ligand compound.

In addition, the present invention provides a catalyst system including the ligand compound or the organochromium compound.

In addition, the present invention provides a method of preparing a linear alpha-olefin, including oligomerizing ethylene in the presence of the catalyst system.

### ADVANTAGEOUS EFFECTS

The organochromium compound and the catalyst system using the same according to the present invention have excellent catalyst activity and shows high selectivity to an alpha-olefin such as 1-hexene and 1-octene, and accordingly, may be usefully used as catalysts for polymerization for preparing a linear alpha-olefin.

### BEST MODE FOR CARRYING OUT THE INVENTION

Hereinafter, the present invention will be explained in more detail to assist the understanding of the present invention.

In the present invention, "oligomerization" means oligomerizing olefins. According to the number of olefins polymerized, the oligomerization is referred to as trimerization or tetramerization, and is collectively referred to as multimerization. Particularly, in the present disclosure, the oligomerization may mean selective preparation of 1-hexene and 1-octene, which are main comonomers of a LLDPE from ethylene.

In the present invention, a "catalyst system" means a state obtainable as a catalyst composition having activity by adding three components including a chromium source, a ligand compound and a cocatalyst, or two components of a transition metal compound and a cocatalyst in an arbitrary order. The three components or two components of the catalyst system may be added in the presence or absence of a solvent and a monomer, and may be used in a supported or unsupported state.

In the case of applying the ligand compound of the present invention to a catalyst system for polymerizing a linear alpha-olefin, excellent catalyst activity may be shown, and selectivity to linear alpha-olefin may be high, and when compared to the conventional PNP-based catalyst, the production amount of a solid polyethylene is small even under the same reaction conditions, and a linear alpha-olefin could be prepared more efficiently.

Particularly, the aniline-based PNP-based organochromium compound of the present invention is mainly utilized for the preparation of a linear alpha-olefin using ethylene, and oligomerization reaction is mainly performed by the reaction under ethylene conditions, to show high selectivity with respect to an alpha-olefin of a liquid type, particularly, 1-hexene or 1-octene of a liquid type. This is because selectivity to an alpha-olefin with a specific length increases through a transition state forming metallacycle in the oligomerization reaction of ethylene.

The ligand compound of the present invention includes a diphosphino aminyl moiety, and aryl having a specific substituent is connected with the terminal of the diphosphino aminyl moiety, and accordingly, the ligand compound may have a type which may play the role of a strong electron donating group.

Due to such structural features, the ligand compound may be applied to a catalyst system for oligomerizing ethylene to show high activity, particularly, high selectivity to 1-hexene, 1-octene, or the like. This is considered due to the interaction between adjacent chromium active points. Particularly, in the case where aryl substituted with a specific substituent is connected with the phosphor (P) atom of diphosphino aminyl, electron density may increase at the phosphor (P) atom and nitrogen (N) atom included in the diphosphino aminyl, and the electrical and three-dimensional properties of the whole ligand compound may change.

Accordingly, there may be changes of the bond between the ligand and the chromium atom, the structure of the catalyst may be stabilized even further, an alpha-olefin may be formed with higher activity and selectivity by changing the energy of a transition state (activation energy) when compared to the conventional metallacycloheptane or metallacyclononane type, and the amount of by-products such as a solid alpha-olefin having a large molecular weight like PE wax, may be reduced even further.

Particularly, the ligand compound of the present invention is characterized in that aryl positioned at the terminal of a diphosphino aminyl residue has a bulky substituent. The bulky substituent prevents the generation of an inactive species by the combination of two molecules of the ligand compound with the chromium atom, and reduces the rotatability of a nitrogen-phosphor bond to prevent the dissociation of the ligand compound in the catalyst, thereby producing a chromium catalyst having high stability and excellent activity and selectivity.

The bulky substituent is bonded at the para position of the aryl. If the substituent is bonded at a meta position or at both positions of meta and para, the steric hindrance of the catalyst may become too excessive, the approach of ethylene may become difficult, and the catalyst activity may be rather deteriorated. Since the ligand compound of the present invention is bonded at the para position, a suitable degree of steric hindrance may be induced, and ethylene may be oligomerized with high activity, while securing catalyst stability.

In addition, to the nitrogen atom bonded to two phosphor atoms, a bulky functional group such as cycloalkyl is bonded, and the bulky substituent bonded to the nitrogen may prevent the rotation of a nitrogen-phosphor bond to maximize the improvement of the catalyst stability and activity.

The ligand compound of the present invention is characterized in being represented by Formula 1 below.

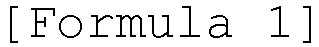

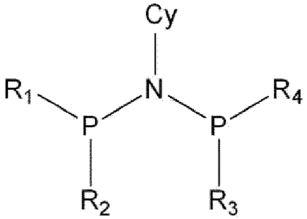

In Formula 1,
Cy is cycloalkyl of 5 to 10 carbon atoms, or cycloalkyl of 5 to 10 carbon atoms, fused with aryl of 6 to 10 carbon atoms, and
R₁ to R₄ are each independently aryl of 6 to 20 carbon atoms, which is para-substituted with a substituent selected from the group consisting of cycloalkyl of 5 to 20 carbon atoms, alkyl of 6 to 20 carbon atoms, alkoxyalkyl of 6 to 20 carbon atoms, arylalkoxyalkyl of 10 to 30 carbon atoms and trialkylsilyl of 3 to 30 carbon atoms.

In the present invention, "cycloalkyl" refers to a nonaromatic cyclic hydrocarbon radical composed of carbon atoms. Non-limiting examples of the cycloalkyl include cyclopropyl, cyclobutyl, cyclopentyl, cyclohexyl and cycloheptyl, without limitation. In the present disclosure, the cycloalkyl includes nonaromatic cyclic hydrocarbon such as bicyclic and tricyclic alkyls, in which two or more nonaromatic cyclic hydrocarbons are fused.

In the present invention, "alkyl" means a hydrocarbon residue of a linear chain, a cyclic or a branched type and includes methyl, ethyl, n-propyl, isopropyl, n-butyl, isobutyl, t-butyl, n-pentyl, isopentyl and hexyl, without limitation.

In the present invention, "alkoxyalkyl" means a substituent in which one or more hydrogen of alkyl are substituted with alkoxy. Particularly, methoxymethyl, ethoxymethyl, methoxyethyl, ethoxyethyl, butoxymethyl, butoxyethyl, butoxypropyl, butoxybutyl, butoxyheptyl, butoxyhexyl, or the like may be included, without limitation.

In the present invention, "arylalkoxyalkyl" means a substituent in which one or more hydrogen of alkyl are substituted with arylakoxy.

In the present invention, "trialkylsilyl" means a substituent represented by -SiR₃ where each R is independently alkyl, and the carbon number of the trialkylsilyl may mean the sum of all the carbon number of R.

In the present invention, "aryl" refers to an arbitrarily substituted benzene ring, or refers to a ring system which may be formed by fusing one or more arbitrary substituents, unless otherwise referred to. Examples of the arbitrary substituent include substituted Cₗ₋₃ alkyl, substituted C₂₋₃ alkenyl, substituted C₂₋₃ alkynyl, heteroaryl, heterocyclic, aryl, arbitrary alkoxy having 1 to 3 fluorine substituents, aryloxy, aralkoxy, acyl, aroyl, heteroaroyl, acyloxy, aroyloxy, heteroaroyloxy, sulfanyl, sulfinyl, sulfonyl, aminosulfonyl, sulfonylamino, carboxyamide, aminocarbonyl, carboxy, oxo, hydroxyl, mercapto, amino, nitro, cyano, halogen or ureido. Such a ring or ring system may be arbitrarily fused with an aryl ring (for example, benzene ring), carbocyclic ring or heterocyclic ring, having arbitrarily one or more substituents. Non-limiting examples include phenyl, naphthyl, tetrahydronaphthyl, biphenyl, indanyl, anthracyl, phenanthryl and substituted derivatives thereof, without limitation.

Cy is cycloalkyl of 5 to 10 carbon atoms, or cycloalkyl of 5 to 10 carbon atoms, fused with aryl of 6 to 10 carbon atoms.

Here, each aryl fused with the cycloalkyl may be unsubstituted or substituted with alkyl of 1 to 8 carbon atoms, particularly, unsubstituted or substituted with alkyl of 1 to 6 carbon atoms, alkyl of 1 to 3 carbon atoms, or methyl.

Particularly, the cycloalkyl of 5 to 10 carbon atoms may be, for example, cyclopentyl, cyclohexyl, cycloheptyl, or cyclooctyl, and the cycloalkyl of 5 to 10 carbon atoms, fused with aryl of 6 to 10 carbon atoms may be fused with one or two aryl of 6 to 10 carbon atoms. For example, phenyl-fused cyclopentyl(dihydroindenyl), phenyl-fused cyclohexyl(tetrahydronaphthyl), phenyl-fused cycloheptyl(6,7,8,9-tetrahydrobenzo[7]annulenyl), phenyl-fused cyclooctyl(5,6,7,8,9,10-hexahydrobenzo[8]annulenyl), or two phenyl-fused cyclopentyl(9H-fluorenyl), may be included.

R₁ to R₄ may be each independently aryl of 6 to 20 carbon atoms, aryl of 6 to 10 carbon atoms, for example, phenyl or naphthyl, preferably, phenyl.

The substituent of R₁ to R₄ may be cycloalkyl of 5 to 20 carbon atoms, cycloalkyl of 6 to 10 carbon atoms, for example, cyclohexyl, cycloheptyl, cyclooctyl, cyclononyl, 1-adamantyl, 2-adamantyl, or the like; alkyl of 6 to 20 carbon atoms, alkyl of 8 to 15 carbon atoms, for example, octyl, nanon-5-yl, 5-methylnonan-5-yl, 5-ethylnonan-5-yl, 5-propylnonan-5-yl, 5-butylnonan-5-yl, or the like; alkoxyalkyl of 6 to 20 carbon atoms, alkoxyalkyl of 9 to 15 carbon atoms, for example, 5-methoxynonan-5-yl, 5-ethoxynonan-5-yl, 5-propoxynonan-5-yl, 5-butoxynonan-5-yl, or the like; arylalkoxyalkyl of 10 to 30 carbon atoms, arylalkoxyalkyl of 14 to 20 carbon atoms, for example, 5-phenylmethoxynonan-5-yl, 5-(2-phenylethoxy)nonan-5-yl, 5-(3-phenylpropoxy)nonan-5-yl, or the like; trialkylsilyl of 3 to 30 carbon atoms, trialkylsilyl of 3 to 15 carbon atoms, trialkylsilyl of 4 to 12 carbon atoms, or trialkylsilyl of 4 to 9 carbon atoms, for example, tributylsilyl, tripropylsilyl, or the like. The carbon number of the trialkylsilyl represents the total carbon number of the trialkylsilyl.

R₁ to R₄ may be the same or different.

The Cy may be any one selected from the group below, but is not limited thereto.

a) 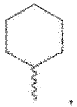 b) 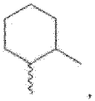 c) 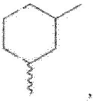 d) 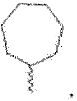 e) 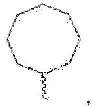 f) 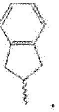 g) 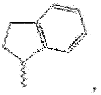 h) 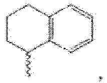 i) 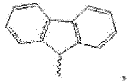
j)

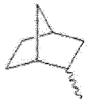

In addition, R₁ to R₄ may be each independently selected from the group below, but is not limited thereto.

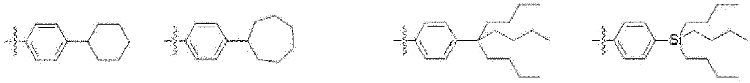

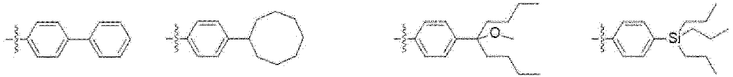

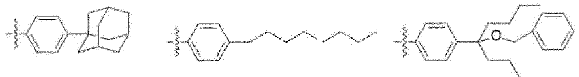

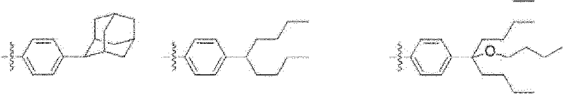

The ligand compound according to the present invention may be accomplished by various combinations in a range satisfying the above-described conditions, in addition to the aforementioned particular embodiments, and all compounds may be applicable as the ligand compound of the present invention as long as they are represented by Formula 1.

In addition, the present invention provides an organochromium compound including the ligand compound represented by Formula 1; and chromium (Cr) coordinated with the ligand compound.

The organochromium compound is the chromium complex compound of the ligand compound and may have a type where the chromium of a chromium source makes coordination bonds with one or more unshared electron pairs of N and two P in the ligand compound represented by Formula 1. That is, in the structure, the phosphor atom or nitrogen atom of a diphosphino aminyl residue provides the chromium atom with an unshared electron pair, and particularly, a bidentated state in which two unshared electron pairs are shared, may be preferable. Such an organochromium compound may be applied to a catalyst system for the polymerization reaction of ethylene to show excellent catalyst activity and high selectivity to 1-hexene and 1-octene.

In addition, the present invention provides a catalyst system including chromium, the ligand compound represented by Formula 1 and a cocatalyst.

The ligand compound represented by Formula 1 may be coordinated with chromium to form an organochromium compound. That is, the catalyst system may be a three-component-based catalyst system including chromium, the ligand compound represented by Formula 1 and a cocatalyst, or a two-component-based catalyst system including the organochromium compound and a cocatalyst.

In the catalyst system, the chromium is derived from a chromium source, and the chromium source is an organic or inorganic chromium compound with an oxidation state of 0 to 6. The chromium source may be, for example, a chromium metal, or a compound in which an arbitrary organic or inorganic radical is bonded to chromium. Here, the organic radical may be alkyl, alkoxy, ester, ketone, amido, carboxylate radical, or the like having 1 to 20 carbon atoms, and the inorganic radical may be halide, sulfate, oxide, or the like.

The chromium source is a compound showing high activity for the oligomerization of an olefin and advantageously used and obtained, and may be one or more compounds selected from the group consisting of chromium(III) acetylacetonate, chromium(III) chloride tetrahydrofuran, chromium(III) 2-ethylhexanoate, chromium(III) acetate, chromium(III) butyrate, chromium(III) pentanoate, chromium(III) laurate, chromium(III) tris(2,2,6,6-tetramethyl-3,5-heptanedionate) and chromium(III) stearate.

In addition, preferably, the cocatalyst is an organometallic compound including a metal in group 13, and any one generally used for polymerizing an olefin in the presence of a transition metal compound may be applied, without specific limitation.

For example, the cocatalyst may be one or more compounds selected from the group consisting of Formulae 2 to 4 below.

[Formula 2] -[Al(Rₐ)-O]ₐ-

In Formula 2, each Rₐ is independently a halogen radical, a hydrocarbyl radical of 1 to 20 carbon atoms, or a halogen-substituted hydrocarbyl radical of 1 to 20 carbon atoms, and
a is an integer of 2 or more.

   [Formula 3] D(R_{b})₃
In Formula 3,
D is aluminum or boron, and
each R_{b} is independently hydrogen, a halogen radical, a hydrocarbyl radical of 1 to 20 carbon atoms, or a halogen-substituted hydrocarbyl radical of 1 to 20 carbon atoms.

   [Formula 4] [L-H]⁺[Z(A)₄]⁻ or [L]⁺[Z(A)₄]-
In Formula 4,
L is a neutral or cationic Lewis acid,
Z is an element in group 13, and
each A is independently an aryl group of 6 to 20 carbon atoms or an alkyl group of 1 to 20 carbon atoms, where one or more hydrogen atoms may be substituted with substituents, and the substituent is halogen, hydrocarbyl of 1 to 20 carbon atoms, alkoxy of 1 to 20 carbon atoms, or aryloxy of 6 to 20 carbon atoms.

   [Formula 5] [(R_{c})ₙL'-H]⁺[B(R_{d})4]⁻
In Formula 5,
L' is N, S or P,
n is an integer of 1 to 3,
each R_{c} is independently hydrogen, hydrocarbyl of 1 to 20 carbon atoms or heterocarbyl, where one or more R_{c} include 6 or more carbon atoms, and a total carbon number of (R_{c})ₙ is greater than 12, and
each R_{d} is independently hydride, dialkylamido, halide, alkoxide, aryloxide, hydrocarbyl, halo-substituted hydrocarbyl radical, halo-substituted alkoxide, halo-substituted aryloxide or halo-substituted aromatic residue having at least one halide on an aromatic residue.

The [(R_{c})L'-H]⁺ is a cation, particularly, a Brønsted acid.

If L' is N or P, the [(R_{c})L'-H]⁺ may be represented by [R_{c1}R_{c2}R_{c3}L₁'-H]+, and if L' is S, the [(R_{c})ₙL'-H]⁺ may be represented by [(R_{c1}R_{c2}L₂')₂-H]+.

Each R_{c} may be independently an aliphatic hydrocarbyl or aliphatic heterohydrocarbyl group, preferably, saturated aliphatic hydrocarbyl or saturated aliphatic heterohydrocarbyl, more preferably, substituted hydrocarbyl or substituted heterohydrocarbyl of which substituent is a nonpolar group, and the substituent of the nonpolar group may include, for example, butyl, pentyl, hexyl, sec-hexyl, cyclohexyl, 2-methylcyclohexyl, 2-ethylcyclohexyl, 2-isopropylcyclohexyl, cyclohexenyl, hexenyl, hexynyl, octyl, cyclo-octyl, cyclooctenyl, 2-ethylhexyl, iso-octyl, decyl, benzyl, phenyl, tolyl, xylyl, o-methylphenyl, o-ethylphenyl, o-isopropylphenyl, o-t-butylphenyl, cumyl, mesityl, biphenyl, naphthyl, anthracenyl, or the like. In addition, R_{c} may include, for example, methyl, ethyl, ethylenyl, propyl, propenyl, propynyl, butyl, pentyl, hexyl, cyclohexyl, 2-methylcyclohexyl, 2-ethylcyclohexyl, octyl, 2-ethylhexyl, iso-octyl, decyl, dodecyl, tetradecyl, octadecyl, 2-isopropylcyclohexyl, benzyl, phenyl, tolyl, xylyl, o-methylphenyl, o-ethylphenyl, o-isopropylphenyl, o-t-butylphenyl, biphenyl, naphthyl, or the like.

In Formula 5, one or more R_{c} may include 6 to 40 carbon atoms and total 13 to 100 carbon atoms, and particularly, one R_{c} may include 6 to 40 carbon atoms and total 21 to 90 carbon atoms.

In Formula 5, R_{d} may be a halo-substituted aromatic residue having at least one halide substituent at an aromatic ring. The halo-substituted aromatic residue may particularly be pentafluorophenyl.

As a method for preparing the catalyst system, firstly, there is provided a preparation method including contacting the organochromium compound with the compound represented by Formula 2 or Formula 3 to prepare a mixture; and adding a compound represented by Formula 4 or 5 to the mixture. Secondly, there is provided a method for preparing a catalyst system by contacting the organochromium compound with the compound represented by Formula 4 or 5. Third, there is provided a preparation method including contacting the organochromium compound with the compound represented by Formula 4 or Formula 5 to prepare a mixture; and adding a compound represented by Formula 2 or 3 to the mixture.

Among the preparation methods of the catalyst system, in the case of the first method or third 0method, the molar ratio of the compound represented by Formula 2 or Formula 3 with respect to the organochromium compound may be 1:2 to 1:5,000, particularly, 1:100 to 1:3,000, more particularly, 1:300 to 1:1,500.

If the molar ratio of the compound represented by Formula 2 or Formula 3 with respect to the organochromium compound is less than 1:2, there may be defects of incompletely performing the alkylation of the organochromium compound, and if the molar ratio is greater than 1:5,000, the alkylation of the organochromium compound may be performed, but there are defects of incompletely performing the activation of the alkylated organochromium compound due to side reactions among the remaining excessive amount of the alkylating agent. In addition, if the molar ratio of the compound represented by Formula 4 or 5 with respect to the organochromium compound is less than 1:1, there are defects of incompletely performing the alkylation of the organochromium compound to deteriorate the activity of the catalyst system, and if the molar ratio is greater than 1:25, the alkylation of the metal compound may be completely performed, but due to the excessive amount of the activating agent remained, there are defects in that the unit cost of the catalyst system may not be economic, or the purity of a polymer produced may be degraded.

In the second method among the preparation methods of the catalyst system, the molar ratio of the compound represented by Formula 4 or 5 with respect to the organochromium compound may be 1:1 to 1:500, particularly, 1:1 to 1:50, more particularly, 1:1 to 1:25. If the molar ratio is less than 1:1, the amount of the activating agent is relatively small, and the activation of the metal compound may be incompletely performed, and there are defects of degrading the activity of the catalyst system. If the molar ratio is greater than 1:500, the activation of the metal compound may be completely performed, but there are defects in that the unit cost of the catalyst system may not be economic, or the purity of a polymer produced may be degraded due to the remaining excessive amount of the activating agent.

As the reaction solvent in preparing the composition, a hydrocarbon-based solvent such as pentane, hexane and heptane, or an aromatic solvent such as benzene and toluene may be used, but is not limited thereto, and all solvents used in this technical field may be used.

In addition, the organochromium compound and the cocatalyst may be used in supported types. As the support, silica or alumina may be used.

The compound represented by Formula 2 is not specifically limited as long as it is alkylaluminoxane. Particular examples may include methylaluminoxane, ethylaluminoxane, isobutylaluminoxane, butylaluminoxane, or the like, more particularly, methylaluminoxane.

The compound represented by Formula 3 is not specifically limited, and particular examples thereof may include trialkyl aluminum, dialkyl aluminum halide, alkyl aluminum dihalide, dialkyl aluminum hydride, alkyl aluminum dihydride, trialkylboron, or the like.

For example, trialkyl aluminum such as trimethylaluminum, triethylaluminum, triisobutylaluminum, tripropylaluminum, tributylaluminum, triisopropylaluminum, tri-s-butylaluminum, tricyclopentylaluminum, tripentylaluminum, triisopentylaluminum, trihexylaluminum, trioctylaluminum, ethyldimethylaluminum, methyldiethylaluminum, triphenylaluminum, and tri-p-tolylaluminum; dialkylaluminum halide such as diethylaluminum chloride; dialkyl aluminum hydride such as diethyl aluminum hydride, di-n-propyl aluminum hydride, diisopropyl aluminum hydride, di-n-butyl aluminum hydride, dibutyl aluminum hydride, diisobutyl aluminum hydride (DIBAH), di-n-octyl aluminum hydride, diphenyl aluminum hydride, di-p-tolyl aluminum hydride, dibenzyl aluminum hydride, phenylethyl aluminum hydride, phenyl-n-propyl aluminum hydride, phenylisopropyl aluminum hydride, phenyl-n-butyl aluminum hydride, phenylisobutyl aluminum hydride, phenyl-n-octyl aluminum hydride, p-tolylethyl aluminum hydride, p-tolyl-n-propyl aluminum hydride, p-tolylisopropyl aluminum hydride, p-tolyl-n-butyl aluminum hydride, p-tolylisobutyl aluminum hydride, p-tolyl-n-octyl aluminum hydride, benzylethyl aluminum hydride, benzyl-n-propyl aluminum hydride, benzylisopropyl aluminum hydride, benzyl-n-butyl aluminum hydride, benzylisobutyl aluminum hydride and benzyl-n-octyl aluminum hydride; alkyl aluminum dihydride such as n-propyl aluminum dihydride, isopropyl aluminum dihydride, n-butyl aluminum dihydride, isobutyl aluminum dihydride, and n-octylaluminum dihydride; and trialkylboron such as trimethylboron, triethylboron, triisobutylboron, tripropylboron and tributylboron, may be used without limitation.

Examples of the compound represented by Formula 4 may include trimethylammonium tetraphenylborate, triethylammonium tetraphenylborate, tripropylammonium tetraphenylborate, tributylammonium tetraphenylborate, N,N-diethylanilinium tetraphenylborate, trimethylammonium tetra(p-tolyl)borate, triethylammonium tetra(p-tolyl)borate, tripropylammonium tetra(p-tolyl)borate, tributylammonium tetra(p-tolyl)borate, N,N-diethylanilinium tetra(p-tolyl)borate, trimethylammonium tetra(o,p-dimethylphenyl)borate, triethylammonium tetra(o,p-dimethylphenyl)borate, tripropylammonium tetra(o,p-dimethylphenyl)borate, tributylammonium tetra(o,p-dimethylphenyl)borate, N,N-diethylanilinium tetra(o,p-dimethylphenyl)borate, trimethylammonium tetrakis(p-trifluoromethylphenyl) borate, triethylammonium tetrakis(p-trifluoromethylphenyl)borate, tripropylammonium tetrakis(p-trifluoromethylphenyl) borate, tributylammonium tetrakis(p-trifluoromethylphenyl)borate, N,N-diethylanilinium tetrakis(p-trifluoromethylphenyl)borate, trimethylammonium tetrakis(pentafluorophenyl)borate, triethylammonium tetrakis(pentafluorophenyl)borate, tripropylammonium tetrakis(pentafluorophenyl)borate, tributylammonium tetrakis(pentafluorophenyl)borate, N,N-diethylanilinium tetrais(pentafluorophenyl)borate, trimethylphosphonium tetraphenylborate, triethylphosphonium tetraphenylborate, tripropylphosphonium tetraphenylborate, tributylphosphonium tetraphenylborate, trimethylcarbonium tetraphenylborate, triethylcarbonium tetraphenylborate, tripropylcarbonium tetraphenylborate, tributylcarbonium tetraphenylborate, trimethylammonium tetraphenylaluminate, triethylammonium tetraphenylaluminate, tripropylammonium tetraphenylaluminate, tributylammonium tetraphenylaluminate, trimethylammonium tetra(p-tolyl)aluminate, triethylammonium tetra(p-tolyl) aluminate, tripropylammonium tetra(p-tolyl)aluminate, tributylammonium tetra(p-tolyl)aluminate, or the like, without limitation.

Examples of the compound represented by Formula 5 may include dihexyl(methyl)ammonium tetrakis(pentafluorophenyl) borate, dioctyl(methyl)ammonium tetrakis(pentafluorophenyl) borate, methyldi(octyl)ammonium tetrakis(pentafluorophenyl) borate, methyldi(methyl)ammonium tetrakis(pentafluorophenyl) borate, dodecyldi(methyl)ammonium tetrakis(pentafluorophenyl) borate, tetradecyldi(methyl)ammonium tetrakis(pentafluorophenyl) borate, hexadecyldi(methyl)ammonium tetrakis(pentafluorophenyl) borate, octadecyldi(methyl)ammonium tetrakis(pentafluorophenyl) borate, eicosyldi(methyl)ammonium tetrakis(pentafluorophenyl) borate, methyldi(decyl)ammonium tetrakis(pentafluorophenyl) borate, methyldi(dodecyl)ammonium tetrakis(pentafluorophenyl) borate, methyldi(tetradecyl)ammonium tetrakis(pentafluorophenyl) borate, methyldi(hexadecyl)ammonium tetrakis(pentafluorophenyl) borate, methyldi(octadecyl)ammonium tetrakis(pentafluorophenyl) borate, methyldi(eicosyl)ammonium tetrakis(pentafluorophenyl) borate, trihexylammonium tetrakis(pentafluorophenyl) borate, trioctylammonium tetrakis(pentafluorophenyl) borate, tri(2-ethylhexyl)ammonium tetrakis(pentafluorophenyl) borate, tri(iso-octyl)ammonium tetrakis(pentafluorophenyl) borate, tridecylammonium tetrakis(pentafluorophenyl) borate, tridodecylammonium tetrakis(pentafluorophenyl) borate, tritetradecylammonium tetrakis(pentafluorophenyl) borate, trihexadecylammonium tetrakis(pentafluorophenyl) borate, trioctadecylammonium tetrakis(pentafluorophenyl) borate, trieicosylammonium tetrakis(pentafluorophenyl) borate, hexyldi(n-butyl)ammonium tetrakis(pentafluorophenyl) borate, octyldi(n-butyl)ammonium tetrakis(pentafluorophenyl) borate, decyldi(n-butyl)ammonium tetrakis(pentafluorophenyl) borate, dodecyldi(n-butyl)ammonium tetrakis(pentafluorophenyl) borate, octadecyldi(n-butyl)ammonium tetrakis(pentafluorophenyl) borate, N,N-dihexylanilinium tetrakis(pentafluorophenyl) borate, N,N-dioctylanilinium tetrakis(pentafluorophenyl) borate, N,N-didodecylanilinium tetrakis(pentafluorophenyl) borate, N-methyl-N-dodecylanilinium tetrakis (pentafluorophenyl) borate, N,N-di(octadecyl)(2,4,6-trimethylanilinium) tetrakis(pentafluorophenyl) borate, cyclohexyldi (dodecyl) ammonium tetrakis(pentafluorophenyl)borate, methyldi (dodecyl) ammonium tetrakis-(2,3,4,6-tetrafluorophenyl) borate, trioctylphosphonium tetrakis(pentafluorophenyl) borate, trihexylphosphonium tetrakis(pentafluorophenyl) borate, tributylphosphonium tetrakis(pentafluorophenyl) borate, dioctyl(methyl)phosphonium tetrakis(pentafluorophenyl) borate, dimethyl(octyl)phosphonium tetrakis(pentafluorophenyl) borate, bis(dihexylsulfide)onium tetrakis(pentafluorophenyl) borate, bis(dioctylsulfide)onium tetrakis(pentafluorophenyl) borate, bis(didecylsulfide)onium tetrakis(pentafluorophenyl) borate, and bis(didodecylsulfide)onium tetrakis(pentafluorophenyl) borate, or the like, without limitation.

Meanwhile, the amount ratio of the components constituting the catalyst system may be determined considering catalyst activity and selectivity to a linear alpha-olefin. According to an embodiment, in the case of the three-component-based catalyst system, the molar ratio of the diphosphino aminyl residue of the ligand compound: chromium source: cocatalyst may preferably be controlled to about 1:1:1 to 10:1:10,000, or about 1:1:100 to 5:1:3,000. In addition, in the case of the two-component-based catalyst system, the molar ratio of the diphosphino aminyl residue of the organochromium compound: cocatalyst may preferably be controlled to 1:1 to 1:10,000, or 1:1 to 1:5,000, or 1:1 to 1:3,000.

Also, the components constituting the catalyst system may be added simultaneously or in an arbitrary order, in the presence or absence of a suitable solvent and monomer, so as to act as a catalyst system having activity. In this case, the suitable solvent may use heptane, toluene, cyclohexane, methylcyclohexane, 1-hexene, 1-octene, diethyl ether, tetrahydrofuran, acetonitrile, dichloromethane, chloroform, chlorobenzene, methanol, acetone, or the like.

In addition, the catalyst system may further include a support. That is, the ligand compound represented by Formula 1 may be applied in a supported state to a support for the oligomerization of ethylene. The support may be a metal, a metal salt or a metal oxide, which may be applied to a general support catalyst. Non-limiting examples of the support may include silica, silica-alumina, silica-magnesia, or the like, and may include a metal oxide, metal carbonate, metal sulfate, or metal nitrate, such as Na₂O, K₂CO₃, BaSO₄, and Mg(NO₃)₂.

Such a catalyst system may preferably be used for the trimerization or tetramerization reaction of ethylene, and may produce 1-hexene or 1-octene with high selectivity by the above-description.

In addition, the present invention provides a method for preparing a linear alpha-olefin, including the step of oligomerizing ethylene in the presence of the catalyst system.

The oligomerization reaction of olefin may be the trimerization or tetramerization reaction of ethylene, and may form 1-hexene or 1-octene as a reaction result.

The method of oligomerizing ethylene according to the present invention may be performed by using ethylene as a raw material and applying the above-described catalyst system, a common apparatus and contacting technique. Non-limiting examples of the oligomerization reaction of ethylene may include homogeneous liquid phase reaction in the presence or absence of an inert solvent, slurry reaction by which a portion or whole of the catalyst system is not dissolved, bulk phase reaction in which an alpha-olefin product acts as a main medium, or gas phase reaction.

The oligomerization reaction of olefin may be performed under an inert solvent. Non-limiting examples of the inert solvent may include benzene, toluene, xylene, cumene, chlorobenzene, dichlorobenzene, heptane, cyclohexane, methylcyclohexane, methylcyclopentane, n-hexane, 1-hexene, 1-octene, or the like.

The polymerization of the olefin may be performed under a temperature of about 0 to about 200°C, or about 0 to about 150°C, or about 30 to about 100°C, or about 50 to about 100°C. In addition, the reaction may be performed under a pressure of about 10.3421 to kPa 20684 kPa (15 to about 3000 psig), or about 10.3421 to 10342 kPa (15 to about 1500 psig), or about 10.3421 to 6895 kPa (15 to about 1000 psig).

### Examples

Hereinafter, the present invention will be explained in more detail referring to the examples. However, the examples are for illustrating the present invention, and the scope of the present invention is not limited thereto.

### [Synthesis of ligand compounds]

Ligand compounds of Synthetic Examples 1 to 17 were prepared according to the reaction below.

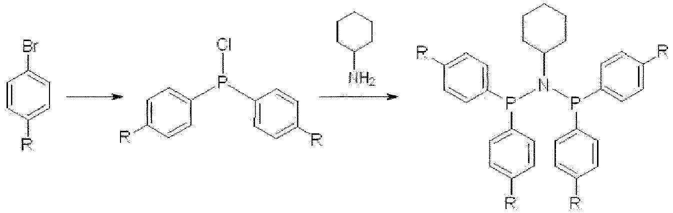

### Synthetic Example 1

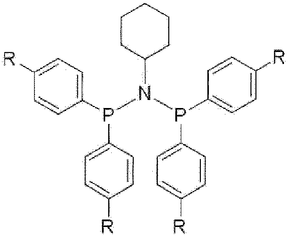

(R = cyclohexyl)

In the reaction, 1-bromo-4-cyclohexylbenzene (2 eq, 20 mmol) of which R is cyclohexyl was dissolved in THF (20 mL) and cooled to -78°C. While keeping the temperature, n-BuLi (2 eq, 20 mmol) was added thereto dropwisely, followed by stirring for 3 hours. Dichloro(diethylamino)phosphine (1 eq, 10 mmol) dissolved in THF (10 mL) was added thereto dropwisely, and the temperature was raised to room temperature, followed by stirring overnight. After removing the solvent in vacuum, the reaction product was dissolved in hexane (30 mL) without additional purification, and hydrochloric acid (2 eq) dissolved in ether was added thereto. After stirring for 15 minutes, filtering was performed, and the filtrate was dried in vacuum.

Then, the compound thus obtained (2.1 eq, 2.1 mmol) was dissolved in DCM (3.8 mL), and TEA (3 eq, 3 mmol) was added thereto. Cyclohexylamine (1 eq, 1 mmol) dissolved in DCM (3.8 mL) was slowly added to a reaction system, followed by stirring at room temperature overnight. After removing the solvent in vacuum, the resultant product was dissolved in hexane (7.6 mL) and loaded on the top of silica. Silica filtration was performed using 1% TEA-added hexane/DCM or 1% TEA-added hexane, and the solution thus obtained was concentrated to obtain a ligand compound.
- N-(bis(4-cyclohexylphenyl)phosphaneyl)-N-cyclohexyl-1,1-bis(4-cyclohexylphenyl)phosphanamine)
- ¹H NMR (500 MHz, C₆D₆) : δ 7.58 (br. s, 8H), δ 7.08 (d, 8H), δ 3.47(pent, 1H), is 2.35(t, 4H), δ 2.21-2.11(m, 2H), δ 1.80(d, 8H), δ 1.71-1.56(m, 16H), δ 1.44-1.11(m, 24H)

### Synthetic Example 2

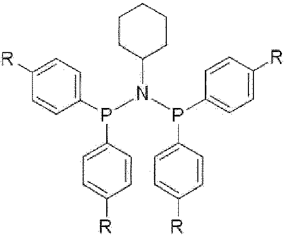

(R = phenyl)

The preparation was performed by the same method as in Synthetic Example 1 except for using a compound in which R is phenyl instead of 1-bromo-4-cyclohexylbenzene (R = cyclohexyl) .
- 1,1-di([1,1'-biphenyl]-4-yl)-N-cyclohexyl-N-(di([1,1'-biphenyl]-4-yl)phosphaneyl)phosphanamine)
- ¹H NMR (500 MHz, C₆D₆) : δ 7.92-7.42 (m, 24H), δ 7.17 (t, 8H), δ 7.10(t, 4H), δ 3.58(pent, 1H), δ 2.28-2.17(m, 2H), δ 1.84-1.77(m, 2H), δ 1.67-1.60(m, 2H), δ 1.48-1.42(m, 1H), δ 1.18-1.07(mm, 3H)

### Synthetic Example 3

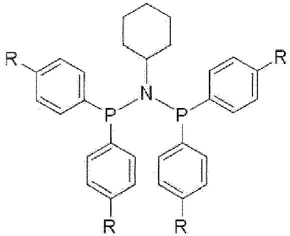

(R = 1-adamantyl)

The preparation was performed by the same method as in Synthetic Example 1 except for using a compound in which R is 1-adamantyl instead of 1-bromo-4-cyclohexylbenzene (R = cyclohexyl).
- 1,1-bis(4-(adamantan-1-yl)phenyl)-N-(bis(4-(adamantan-1-yl)phenyl)phosphaneyl)-N-cyclohexylphosphanamine)
- ¹H NMR (500 MHz, C₆D₆) : δ 7.52(br. s, 8H), δ 6.99(d, 8H), δ 3.43(pent, 1H), δ 2.43-2.36(m, 24H), δ 2.26-2.10(m, 14H), δ 1.85-1.74(m, 26H), δ 1.65-1.58(m, 2H), δ 1.46-1.40(m, 1H), δ 1.16-1.05(m, 3H)

### Synthetic Example 4

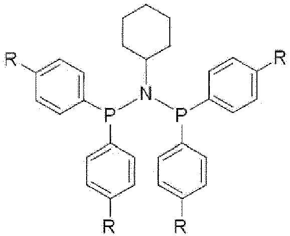

(R = 2-adamantyl)

The preparation was performed by the same method as in Synthetic Example 1 except for using a compound in which R is 2-adamantyl instead of 1-bromo-4-cyclohexylbenzene (R = cyclohexyl).
- 1,1-bis(4-(adamantan-2-yl)phenyl)-N-(bis(4-(adamantan-2-yl)phenyl)phosphaneyl)-N-cyclohexylphosphanamine)
- ¹H NMR (500 MHz, C₆D₆) : δ 7.56(br. s, 8H), δ 7.05(d, 8H), δ 3.45(pent, 1H), δ 3.18(t, 4H), δ 2.38-2.32(m, 8H), δ 2.30-2.17(m, 10H), δ 1.85-1.72(m, 42H), δ 1.67-1.60(m, 2H), δ 1.48-1.42(m, 1H), δ 1.18-1.07(m, 3H)

### Synthetic Example 5

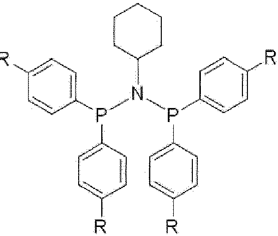

(R = cycloheptyl)

The preparation was performed by the same method as in Synthetic Example 1 except for using a compound in which R is cycloheptyl instead of 1-bromo-4-cyclohexylbenzene (R = cyclohexyl).
- N-(bis(4-cycloheptylphenyl)phosphaneyl)-N-cyclohexyl-1,1-bis(4-cyclooctylphenyl)phosphanamine)
- ¹H NMR (500 MHz, C₆D₆) : δ 7.57 (br. s, 8H), δ 7.07(d, 8H), δ 3.46(pent, 1H), δ 2.34(t, 4H), δ 2.28-2.17(m, 2H), δ 1.86-1.76(m, 10H), δ 1.69-1.58(m, 34H), δ 1.50-1.41(m, 9H), δ 1.18-1.07(mm, 3H)

### Synthetic Example 6

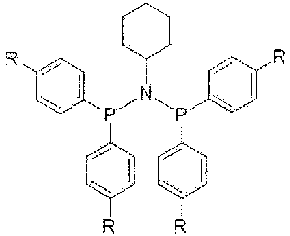

(R = cyclooctyl)

The preparation was performed by the same method as in Synthetic Example 1 except for using a compound in which R is cyclooctyl instead of 1-bromo-4-cyclohexylbenzene (R = cyclohexyl).
- N-(bis(4-cyclooctylphenyl)phosphaneyl)-N-cyclohexyl-1,1-bis(4-cyclooctylphenyl)phosphanamine)
- ¹H NMR (500 MHz, C₆D₆) : δ 7.57 (br. s, 8H), δ 7.07(d, 8H), δ 3.45(pent, 1H), δ 2.34 (t, 4H), δ 2.27-2.16(m, 2H), δ 1.85-1.75(m, 10H), δ 1.70-1.56(m, 42H), δ 1.51-1.41(m, 9H), δ 1.19-1.06(m, 3H)

### Synthetic Example 7

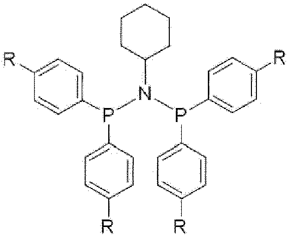

(R = octyl)

The preparation was performed by the same method as in Synthetic Example 1 except for using a compound in which R is octyl instead of 1-bromo-4-cyclohexylbenzene (R = cyclohexyl) .
- N-(bis(4-octylphenyl)phosphaneyl)-N-cyclohexyl-1,1-bis(4-octylphenyl)phosphanamine)
- ¹H NMR (500 MHz, C₆D₆) : δ 7.62(br. s, 8H), δ 7.10(d, 8H), δ 3.50(pent, 1H), δ 2.64(t, 8H), δ 2.28-2.17(m, 2H), δ 1.95-1.75(m, 42H), δ 1.67-1.60(m, 2H), δ 1.48-1.42(m, 1H), δ 1.18-1.07(m, 3H), δ 0.77(t, 12H)

### Synthetic Example 8

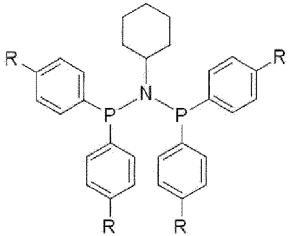

(R = nonan-5-yl)

The preparation was performed by the same method as in Synthetic Example 1 except for using a compound in which R is nonan-5-yl instead of 1-bromo-4-cyclohexylbenzene (R = cyclohexyl).
- N-(bis(4-(nonan-5-yl)phenyl)phosphaneyl)-N-cyclohexyl-1,1-bis(4-(nonan-5-yl)phenyl)phosphanamine)
- ¹H NMR (500 MHz, C₆D₆) : δ 7.58(br. s, 8H), δ 7.09(d, 8H), δ 3.57(pent, 1H), δ 2.38(pent, 4H), δ 2.28-2.17(m, 2H), δ 1.84-1.77(m, 2H), δ 1.73-1.64(m, 8H), δ 1.67-1.60(m, 2H), δ 1.57-1.49(m, 8H), δ 1.48-1.42(m, 1H), δ 1.19-1.02(m, 27H), δ 1.00-0.89(m, 8H), δ 0.77(t, 24H)

### Synthetic Example 9

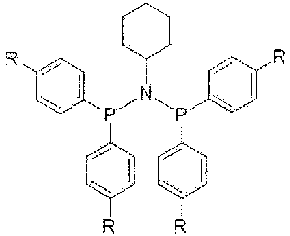

(R = 5-butyl-nonan-5-yl)

The preparation was performed by the same method as in Synthetic Example 1 except for using a compound in which R is 5-butyl-nonan-5-yl instead of 1-bromo-4-cyclohexylbenzene (R = cyclohexyl).
- N-(bis(4-(5-butylnonan-5-yl)phenyl)phosphaneyl)-1,1-bis(4-(5-butylnonan-5-yl)phenyl)-N-cyclohexylphosphanamine)
- ¹H NMR (500 MHz, C₆D₆) : δ 7.51(br. s, 8H), δ 7.00(d, 8H), δ 3.43(pent, 1H), δ 2.28-2.17(m, 2H), δ 1.84-1.77(m, 2H), δ 1.73-1.64(m, 12H), δ 1.67-1.60(m, 2H), δ 1.57-1.49(m, 12H), δ 1.48-1.42(m, 1H), δ 1.19-1.02(m, 31H), δ 1.00-0.89(m, 8H), δ 0.77(t, 36H)

### Synthetic Example 10

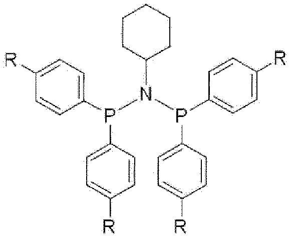

(R = tributylsilyl)

The preparation was performed by the same method as in Synthetic Example 1 except for using a compound in which R is tributylsilyl instead of 1-bromo-4-cyclohexylbenzene (R = cyclohexyl).
- ¹H NMR(500 MHz, C₆D₆) : δ 8.07-7.38(m, 16H), δ 3.40 (pent, 1H), δ 2.15-2.01(m, 2H), δ 1.60-1.49(m, 4H), δ 1.40-1.29(m, 48H), δ 1.05-0.97(m, 2H), δ 0.93-0.85(m, 38H), δ 0.84-0.77(m, 24H)

### Synthetic Example 11

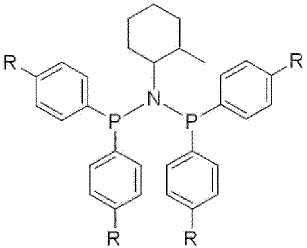

(R = tributylsilyl)

The preparation was performed by the same method as in Synthetic Example 1 except for using a compound in which R is tributylsilyl instead of 1-bromo-4-cyclohexylbenzene (R = cyclohexyl) and using 2-methylcyclohexaneamine instead of cyclohexaneamine.
- ¹H NMR (500 MHz, C₆D₆): δ 8.05-7.36(m, 16H), δ 3.30(q, 1H), δ 2.13-2.00(m, 2H), δ 1.59-1.50(m, 3H), δ 1.41-1.28(m, 48H), δ 1.04-0.96(m, 2H), δ 0.94-0.84(m, 41H), δ 0.83-0.75(m, 24H)

### Synthetic Example 12

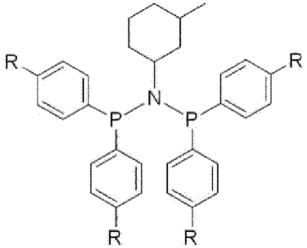

(R = tributylsilyl)

The preparation was performed by the same method as in Synthetic Example 1 except for using a compound in which R is tributylsilyl instead of 1-bromo-4-cyclohexylbenzene (R = cyclohexyl) and using 3-methylcyclohexaneamine instead of cyclohexaneamine.
- ¹H NMR(500 MHz, C₆D₆): δ 8.06-7.38(m, 16H), δ 3.38(pent, 1H), δ 2.14-2.01(m, 2H), δ 1.61-1.48(m, 4H), δ 1.41-1.29(m, 48H), δ 1.04-0.95(m, 2H), δ 0.94-0.85(m, 40H), δ 0.84-0.76(m, 24H)

### Synthetic Example 13

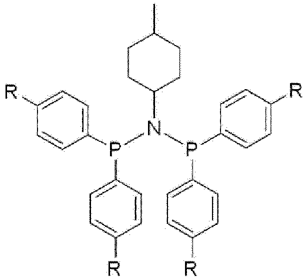

(R = tributylsilyl)

The preparation was performed by the same method as in Synthetic Example 1 except for using a compound in which R is tributylsilyl instead of 1-bromo-4-cyclohexylbenzene (R = cyclohexyl) and using 4-methylcyclohexaneamine instead of cyclohexaneamine.
- ¹H NMR (500 MHz, C₆D₆): δ 8.07-7.38(m, 16H), δ 3.39(pent, 1H), δ 2.15-2.01(m, 2H), δ 1.65-1.49(m, 3H), δ 1.40-1.29(m, 48H), δ 1.05-0.96(m, 2H), δ 0.93-0.85(m, 41H), δ 0.84-0.75(m, 24H)

### Synthetic Example 14

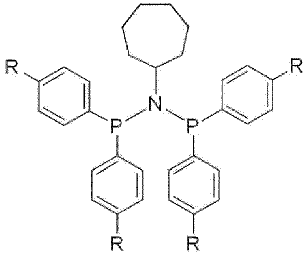

(R = tributylsilyl)

The preparation was performed by the same method as in Synthetic Example 1 except for using a compound in which R is tributylsilyl instead of 1-bromo-4-cyclohexylbenzene (R = cyclohexyl) and using cycloheptaneamine instead of cyclohexaneamine.
- ¹H NMR (500 MHz, C₆D₆): δ 8.06-7.35(m, 16H), δ 3.37(pent, 1H), δ 2.15-2.01(m, 2H), δ 1.62-1.47(m, 6H), δ 1.40-1.29(m, 48H), δ 1.05-0.97(m, 2H), δ 0.93-0.85(m, 38H), δ 0.84-0.77(m, 24H)

### Synthetic Example 15

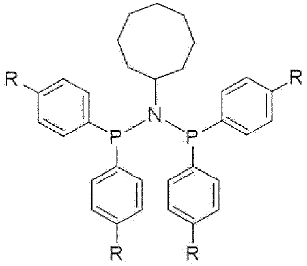

(R = tributylsilyl)

The preparation was performed by the same method as in Synthetic Example 1 except for using a compound in which R is tributylsilyl instead of 1-bromo-4-cyclohexylbenzene (R = cyclohexyl) and using cyclooctaneamine instead of cyclohexaneamine.
- ¹H NMR (500 MHz, C₆D₆): δ 8.07-7.34(m, 16H), δ 3.37(pent, 1H), δ 2.14-2.00(m, 2H), δ 1.62-1.47(m, 6H), δ 1.40-1.29(m, 48H), δ 1.07-0.95(m, 4H), δ 0.93-0.85(m, 38H), δ 0.84-0.77(m, 24H)

### Synthetic Example 16

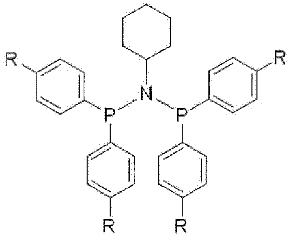

(R = tripropylsilyl)

The preparation was performed by the same method as in Synthetic Example 1 except for using a compound in which R is tripropylsilyl instead of 1-bromo-4-cyclohexylbenzene (R = cyclohexyl).
- ¹H NMR (500 MHz, C₆D₆) : δ 8.09-7.39(m, 16H), δ 3.42 (pent, 1H), δ 2.17-2.02(m, 2H), δ 1.61-1.50(m, 4H), δ 1.42-1.30(m, 24H), δ 1.06-0.97(m, 2H), δ 0.93-0.86(m, 38H), δ 0.85-0.77 (m, 24H)

### Synthetic Example 17

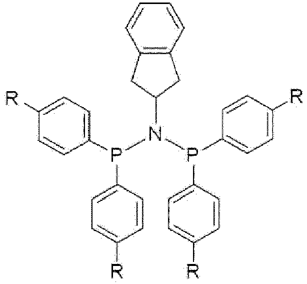

(R = tributylsilyl)

The preparation was performed by the same method as in Synthetic Example 1 except for using a compound in which R is tributylsilyl instead of 1-bromo-4-cyclohexylbenzene (R = cyclohexyl) and using 2,3-dihydro-1H-indene-ylamine instead of cyclohexaneamine.
- ¹H NMR (500 MHz, C₆D₆) : δ 8.07-7.38(m, 18H), δ 7.13(d, 2H), δ 3.53(pent, 1H), δ 2.52-2.41(m, 2H), δ 2.01-1.88(m, 2H), δ 1.40-1.29(m, 48H), δ 0.93-0.85(m, 36H), δ 0.84-0.77(m, 24H)

### Synthetic Example 18

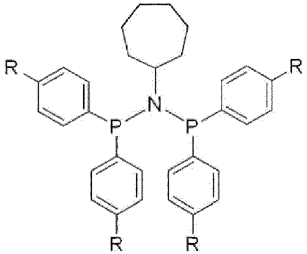

(R = tripropylsilyl)

The preparation was performed by the same method as in Synthetic Example 1 except for using a compound in which R is tripropylsilyl instead of 1-bromo-4-cyclohexylbenzene (R = cyclohexyl) and using cycloheptaneamine instead of cyclohexaneamine.

¹H NMR (500 MHz, C₆D₆) : δ 8.20-7.65(br, 4H), δ 7.60-7.15(br, 4H), δ 7.45(s, 8H), δ 3.69(pent, 1H), δ 2.30(m, 2H), δ 1.90-1.80(m, 2H), δ 1.60-1.50(m, 2H), δ 1.48-1.20(m, 40H), δ 0.98(t, 36H), δ 0.77(m, 24H)

### Synthetic Example 19

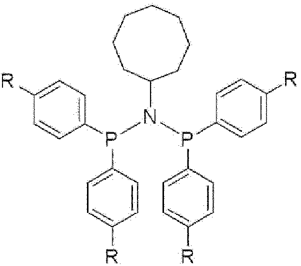

(R = tripropylsilyl)

The preparation was performed by the same method as in Synthetic Example 1 except for using a compound in which R is tripropylsilyl instead of 1-bromo-4-cyclohexylbenzene (R = cyclohexyl) and using cyclooctaneamine instead of cyclohexaneamine.

¹H NMR (500 MHz, C₆D₆) : δ 8.15-7.70 (br, 4H), δ 7.45(d, 8H), δ 7.70-7.45 (br, 4H), δ 3.81 (pent, 1H), δ 2.43-2.28 (m, 2H), δ 1.88-1.75(m, 2H), δ 1.62-1.55(m, 2H), δ 1.55-1.48(m, 2H), δ 1.48-1.29(m, 38H), δ 0.97(t, 40H), δ 0.81-0.72(m, 26H)

### Synthetic Example 20

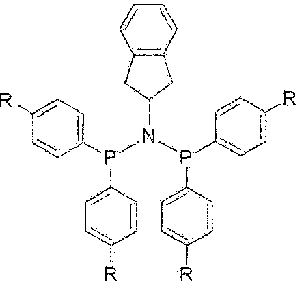

(R = tripropylsilyl)

The preparation was performed by the same method as in Synthetic Example 1 except for using a compound in which R is tripropylsilyl instead of 1-bromo-4-cyclohexylbenzene (R = cyclohexyl) and using 2,3-dihydro-1H-indene-2-amine instead of cyclohexaneamine.
- ¹H NMR (500 MHz, C₆D₆) : δ 7.70-7.25 (m, 16H), δ 7.05-6.95 (m, 4H), δ 4.68-4.54 (m, 1H), δ 3.72-3.62 (m, 2H), δ 2.74-2.68(m, 2H), δ 1.42-1.32(m, 24H), δ 1.01-0.94 (m, 36H), δ 0.79-0.73 (m, 24H)

### Synthetic Example 21

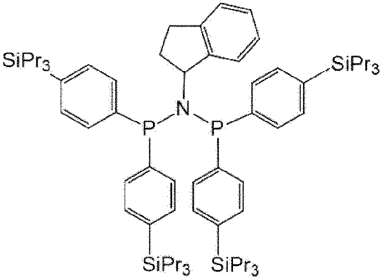

(SiPr₃ = tripropylsilyl)

The preparation was performed by the same method as in Synthetic Example 20 except for using 2,3-dihydro-1*H*-indene-1-amine instead of 2,3-dihydro-1*H*-indene-2-amine.
- ¹H NMR (500 MHz, C₆D₆): δ 7.92-7.62 (m, 4H), δ 7.50-6.35 (m, 12H), δ 7.09-7.35 (m, 12H), δ 7.09-7.06 (m, 2H), δ 7.00-6.95(m, 1H), δ 6.48 (d, 1H), δ 5.40-5.28 (m, 1H), δ 2.53-2.40 (m, 2H), δ 2.11-2.03 (m, 1H), δ 1.42-1.30 (m, 25H), δ 1.00-0.92 (m, 36H), δ 0.80-0.70 (m, 24H)

### Synthetic Example 22

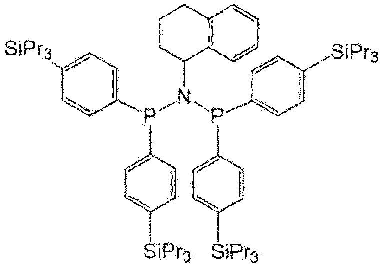

(SiPr₃ = tripropylsilyl)

The preparation was performed by the same method as in Synthetic Example 20 except for using 1,2,3,4-tetrahydronaphthalene-1-amine instead of 2,3-dihydro-1H-indene-2-amine.
- ¹H NMR(500 MHz, C₆D₆) : δ 8.00-7.30 (m, 18H), δ 7.03-6.91 (m, 3H), δ 5.02 (q, 1H), δ 2.76-2.66 (m, 1H), δ 2.53-2.45 (m, 1H), δ 2.33-2.20 (m, 1H), δ 1.62-1.53 (m, 1H), δ 1.50-1.20 (m, 24H), δ 1.05-0.85 (m, 36H), δ 0.83-0.73 (m, 14H), δ 0.73-0.68 (m, 12H)

### Synthetic Example 23

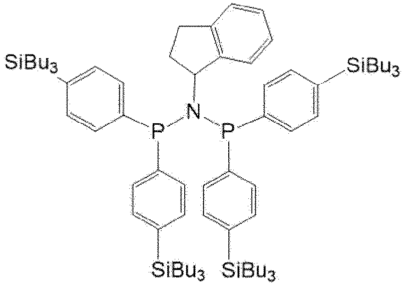

(SiBu₃ = tributylsilyl)

The preparation was performed by the same method as in Synthetic Example 1 except for using a compound in which R is tributylsilyl instead of 1-bromo-4-cyclohexylbenzene (R = cyclohexyl) and using 2,3-dihydro-1*H*-indene-1-amine instead of cyclohexaneamine.
- ¹H NMR (500 MHz, C₆D₆) : δ 8.06-7.92 (m, 4H), δ 7.48-7.36(m, 12H), δ 7.11-7.08 (m, 2H), δ 7.01-6.98 (m, 1H), δ 6.63 (d, 1H), δ 5.72-5.58 (m, 1H), δ 2.53-2.40 (m, 2H), δ 2.11-2.03(m, 1H), δ 1.48-1.36(m, 48H), δ 1.04-0.97(m, 36H), δ 0.86-0.70 (m, 24H)

### Synthetic Example 24

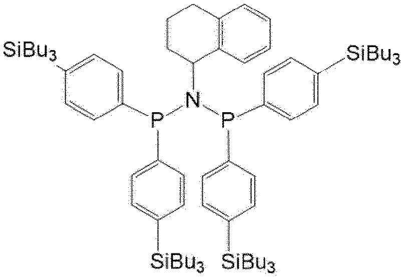

(SiBu₃ = tributylsilyl)

The preparation was performed by the same method as in Synthetic Example 23 except for using 1,2,3,4-tetrahydronaphthalene-1-amine instead of 2,3-dihydro-1H-indene-1-amine.
- ¹H NMR(500 MHz, C₆D₆) : δ 8.01-7.29 (m, 18H), δ 7.12-6.99(m, 3H), δ 5.05 (q, 1H), δ 2.98-2.77 (m, 1H), δ 2.43-2.32 (m, 1H), δ 2.30-2.27 (m, 1H), δ 1.62-1.53 (m, 1H), δ 1.52-1.23 (m, 48H), δ 1.08-0.89 (m, 36H), δ 0.81-0.70 (m, 13H), δ 0.66-0.64(m, 12H)

In addition, the ligand compounds of Synthetic Examples 25 to 27 were prepared according to the reaction below.

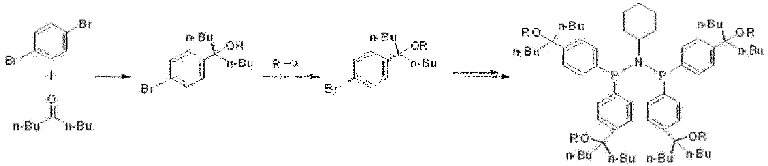

### Synthetic Example 25

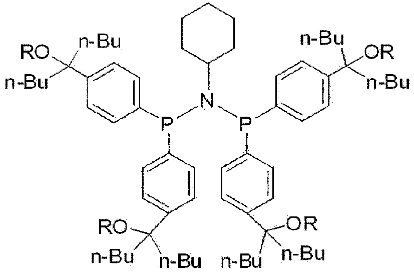

(R = methyl)

p-dibromobenzene (1 eq, 40 mmol) was dissolved in ether (100 mL) and cooled to -78°C. n-BuLi (1.05 eq, 42 mmol) was slowly injected, and the temperature of the reactants was raised to room temperature, followed by stirring for 2 hours. The reaction product was cooled to -78°C, and 5-nonanone (1 eq, 40 mmol) was slowly injected thereto. The temperature of the reaction product was raised to room temperature, and stirring was performed overnight. After quenching with a NH₄Cl saturated aqueous solution, extraction with DCM was performed. Moisture was removed using MgSO₄, filtering was performed to remove a solid, and the solvent was removed under a reduced pressure. Through purification by silica column chromatography, a liquid compound was obtained.

To a suspension in which NaH (1.2 eq, 12 mmol) was dispersed in THF (31 mL), a solution in which the above compound (1 eq, 10 mmol) was dissolved in THF (93 mL) was slowly added. The reactants were stirred and refluxed for 2 hours. After cooling to room temperature, CH3-X (1.2 eq, 12 mmol; X = I, Br, Cl, OTf, etc.) where R is methyl in the Reaction was injected thereto, followed by stirring overnight. The solvent was removed under a reduced pressure, and through purification by silica column chromatography, 1-bromo-4-(5-methoxynonan-5-yl)benzene was obtained.

1-bromo-4-(5-methoxynonan-5-yl)benzene (2 eq, 20 mmol) was dissolved in THF (20 mL) and cooled to -78°C. While maintaining the temperature, n-BuLi (2 eq, 20 mmol) was added thereto dropwisely, and stirring was performed for 3 hours. Dichloro (diethylamino) phosphine (1 eq, 10 mmol) dissolved in THF (10 mL) was added thereto dropwisely, the temperature was raised to room temperature, and stirring was performed overnight. After removing the solvent in vacuum, the resultant product was dissolved in hexane (30 mL) without purification, and hydrochloric acid (2 eq) dissolved in ether was added thereto. After stirring for 15 minutes, filtering was performed, and the filtrate was dried in vacuum.

Then, the compound thus obtained (2.1 eq, 2.1 mmol) was dissolved in DCM (3.8 mL), and TEA (3 eq, 3 mmol) was added thereto. Cyclohexylamine (1 eq, 1 mmol) dissolved in DCM (3.8 mL) was slowly added to a reaction system, followed by stirring at room temperature overnight. After removing the solvent in vacuum, the resultant product was dissolved in hexane (7.6 mL) and loaded on the top of silica. Silica filtration was performed using 1% TEA-added hexane/DCM (1:1), and the solution thus obtained was concentrated to obtain a ligand compound.
- N-(bis(4-(5-methoxynonan-5-yl)phenyl)phosphaneyl)-N-cyclohexyl-1,1-bis(4-(5-methoxynonan-5-yl)phenyl)phosphanamine)
- ¹H NMR (500 MHz, C₆D₆) : δ 7.58(br. s, 8H), δ 7.08 (d, 8H), δ 3.58(pent, 1H), δ 2.90(s, 12H), δ 2.28-2.17(m, 2H), δ 1.84-1.77(m, 2H), δ 1.73-1.68(m, 8H), δ 1.67-1.60(m, 2H), δ 1.56-1.49(m, 8H), δ 1.48-1.42(m, 1H), δ 1.18-1.03(m, 27H), δ 1.00-0.91(m, 8H), δ 0.77(t, 24H)

### Synthetic Example 26

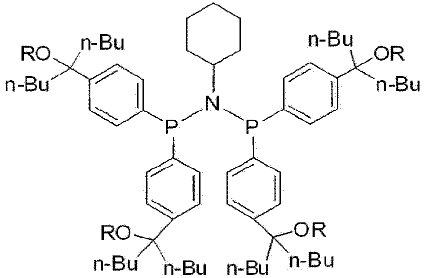

(R = benzyl)

The same method was performed as in Synthetic Example 25 except for using a compound where R is benzyl instead of CH₃-X (R = methyl) .
- 1,1-bis(4-(5-(benzyloxy)nonan-5-yl)phenyl)-N-(bis(4-(5-(benzyloxy)nonan-5-yl)phenyl)phosphaneyl)-N-cyclohexylphosphanamine)
- ¹H NMR (500 MHz, C₆D₆) : δ 7.60-7.32(m, 28H), δ 7.08(d, 8H), δ 3.58(pent, 1H), δ 3.20(s, 8H), δ 2.28-2.17(m, 2H), δ 1.84-1.77(m, 2H), δ 1.73-1.68(m, 8H), δ 1.67-1.60(m, 2H), δ 1.56-1.49(m, 8H), δ 1.48-1.42(m, 1H), δ 1.18-1.03(m, 27H), δ 1.00-0.91(m, 8H), δ 0.77(t, 24H)

### Synthetic Example 27

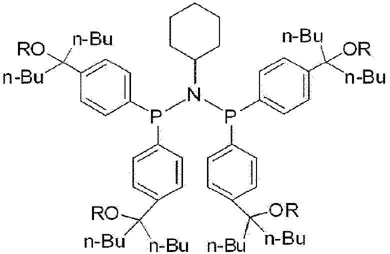

(R = butyl)

The same method was performed as in Synthetic Example 25 except for using a compound where R is butyl instead of CH₃-X (R = methyl) .
- N-(bis(4-(5-butoxynonan-5-yl)phenyl)phosphaneyl)-1,1-bis(4-(5-butoxynonan-5-yl)phenyl)-N-cyclohexylphosphanamine)
- ¹H NMR (500 MHz, C₆D₆) : δ 7.57 (br. s, 8H), δ 7.07(d, 8H), δ 3.58(pent, 1H), δ 3.02(t, 8H), δ 2.28-2.17(m, 2H), δ 1.85-1.78(m, 10H), δ 1.74-1.59(m, 18H), δ 1.56-1.49(m, 8H), δ 1.48-1.42(m, 1H), δ 1.18-1.03(m, 27H), δ 1.00-0.91(m, 8H), δ 0.80-0.75(m, 36H)

### Comparative Synthetic Example 1

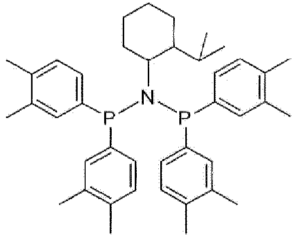

Under argon, 2-isopropylcyclohexan-1-amine (10 mmol) and triethylamine (3 eq. to amine) were dissolved in dichloromethane (80 mL). In a state of immersing a flask into a water bath, chlorobis(3,4-dimethylphenyl)phosphine (20 mmol) was slowly added, and then, stirred overnight. After removing the solvent in vacuum, another solvent of diethyl ether, tetrahydrofuran or hexane was injected, stirring was sufficiently performed, and a triethylammonium chloride salt was removed using an air-free glass filter. The solvent was removed from the filtrate to obtain a product.
- ³¹P NMR(202 MHz, CDCl₃): δ 54.5(br s)

### Comparative Synthetic Example 2

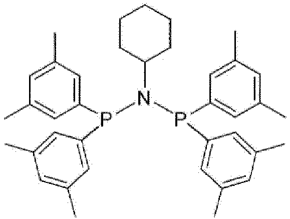

Chlorobis(3,5-dimethylphenyl)phosphine (2.1 eq, 2.1 mmol) was dissolved in DCM (3.8 mL), and TEA (3 eq, 3 mmol) was added. Cyclohexaneamine (1 eq, 1 mmol) dissolved in DCM (3.8 mL) was slowly added to a reaction system, followed by stirring at room temperature overnight. After removing the solvent in vacuum, the resultant product was dissolved in hexane (7.6 mL) and loaded on the top of silica. Silica filtration was performed using 1% TEA-added hexane/DCM (1:1), and the solution thus obtained was concentrated to obtain a ligand compound.

### Comparative Synthetic Example 3

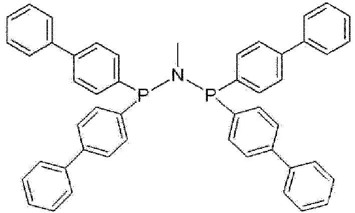

The same method was performed as in Synthetic Example 1 except for using a compound where R is phenyl instead of 1-bromo-4-cyclohexylbenzene (R = cyclohexyl) and using methenamine instead of cyclohexaneamine.

### Comparative Synthetic Example 4

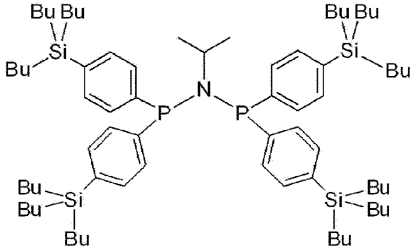

(Bu = n-butyl)

The same method was performed as in Synthetic Example 1 except for using a compound where R is tributylsilyl instead of 1-bromo-4-cyclohexylbenzene (R = cyclohexyl) and using isopropylamine instead of cyclohexaneamine.

### Comparative Synthetic Example 5

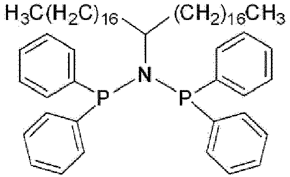

Chlorodiphenylphosphine (2.1 eq, 2.1 mmol) was dissolved in DCM (3.8 mL), and TEA (3 eq, 3 mmol) was added. An amine compound below (1 eq, 1 mmol) dissolved in DCM (3.8 mL) was slowly added to a reaction system, followed by stirring at room temperature overnight. After removing the solvent in vacuum, the resultant product was dissolved in hexane (7.6 mL) and loaded on the top of silica. Silica filtration was performed using 1% TEA-added hexane/DCM (1:1), and the solution thus obtained was concentrated to obtain a ligand compound.

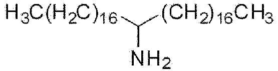

¹H NMR(500 MHz, C₆D₆): δ 7.50-6.70 (br s, 2H), δ 3.03 (apparent quintet, *J*=6.3 Hz, 1H), δ 1.62-1.05 (m, 64H), δ 0.88 (t, *J*=6.6 Hz, 6H)

### Comparative Synthetic Example 6

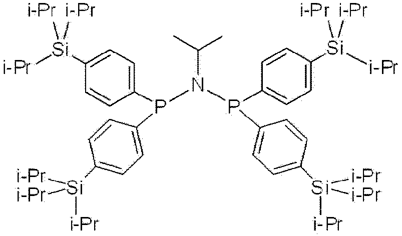

### (i-Pr = isopropyl)

The same method was performed as in Synthetic Example 1 except for using a compound where R is triisopropylsilyl instead of 1-bromo-4-cyclohexylbenzene (R = cyclohexyl) and using isopropylamine instead of cyclohexaneamine.

¹H NMR (500 MHz, C₆D₆): δ 8.12-7.26 (br, 8H), δ 7.45 (d, J=6.6 Hz, 8H), δ 3.83 (m, 1H, NCH), δ 1.32 (m, 12H, SiCH), δ 1.23 (d, *J*=6.6 Hz, 6H, NCHCH₃), δ 1.10 ppm (t, *J*=7.8 Hz, 72H, CH₃)

### Comparative Synthetic Example 7

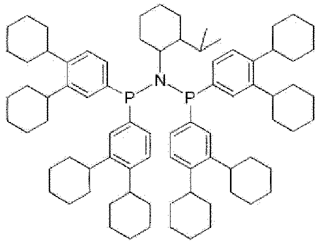

The same method was performed as in Synthetic Example 1 except for using chlorobis(3,4-dicyclohexylphenyl)phosphine instead of chlorobis(3,4-dimethylphenyl)phosphine.

### Comparative Synthetic Example 8

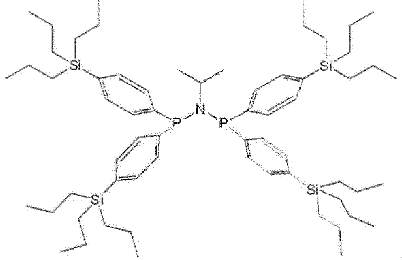

The same method was performed as in Synthetic Example 1 except for using a compound where R is tripropylsilyl instead of 1-bromo-4-cyclohexylbenzene (R = cyclohexyl) and using tripropylamine instead of cyclohexaneamine.

¹H NMR (500 MHz, C₆D₆): δ 8.16-7.20 (br, 8H), δ 7.46 (d, J=6.8 Hz, 8H), δ 3.87 (m, 1H, NCH), δ 1.42-1.33 (br, 48H), δ 1.27 (d, *J*=6.0 Hz, 6H, NCHCH₃), δ 0.98 (t, *J*=7.8 Hz, 36H, CH₃), δ 0.74 ppm (q, *J*=7.2 Hz, 48H, SiCH₂)

### [Manufacture of catalyst system and process of ethylene oligomerization reaction]

### Example 1a

Under an argon gas atmosphere, chromium(III) acetylacetonate (Cr(acac)₃, 17.5 mg, 0.05 mmol) and the ligand compound (0.010 mmol) according to Synthetic Example 1 were put in a flask, and methylcyclohexane (100 mL) was added and stirred to prepare a catalyst solution of 5 mM (based on Cr).

A Parr reactor with a volume of 600 mL was prepared, vacuum was applied at 120°C for 2 hours, the inside was replaced with argon, and the temperature was reduced to 80°C. Then, cyclohexane (180 mL) and 2 mL of MMAO (isoheptane solution, Al/Cr = 600) were injected, and the catalyst solution (2 mL, 1.0 µmol Cr) was injected thereto. After stirring at 500 rpm for 2 minutes, the valve of an ethylene line adjusted to 30 bar was opened to fill the inside of the reactor with ethylene, and then, stirring was performed at 500 rpm for 15 minutes. The ethylene line valve was closed, the reactor was cooled to 0°C using a dry ice/acetone bath, unreacted ethylene was slowly ventilated, and 0.5 mL of nonane (GC internal standard) was injected. After stirring for 10 seconds, 2 mL of a liquid part of the reactor was taken and quenched with water, and an organic part thus obtained was filtered using a PTFE syringe filter to manufacture a GC-FID sample.

Then, the distribution of a liquid product was analyzed by GC (Agilent Co., 6890N, Alltech AT-5 (30 m × 0.32 mm ID × 0.25 µm; series no. 12446)). To a remaining solution, 400 mL of ethanol/HCl (10 vol% of aqueous 12 M HCl solution) was added, stirring and filtering were performed, and the amount of a solid product was analyzed. The polymer thus obtained was dried in a vacuum oven of 65°C overnight.

### Examples 2a to 10a, and 25a to 27a, and Comparative Examples 1a to 3a, and 7a

The same method as in Example 1a was performed except for changing the type of the catalyst as in Table 1 below.

**[Table 1]**

| | Catalyst | Cocatalyst | Chromium source |
|---|---|---|---|
| Example 1a | Synthetic Example 1 | MMAO | Cr(acac)₃ |
| Example 2a | Synthetic Example 2 | MMAO | Cr(acac)₃ |
| Example 3a | Synthetic Example 3 | MMAO | Cr(acac)₃ |
| Example 4a | Synthetic Example 4 | MMAO | Cr(acac)₃ |
| Example 5a | Synthetic Example 5 | MMAO | Cr(acac)₃ |
| Example 6a | Synthetic Example 6 | MMAO | Cr(acac)₃ |
| Example 7a | Synthetic Example 7 | MMAO | Cr(acac)₃ |
| Example 8a | Synthetic Example 8 | MMAO | Cr (acac)₃ |
| Example 9a | Synthetic Example 9 | MMAO | Cr(acac)₃ |
| Example 10a | Synthetic Example 10 | MMAO | Cr(acac)₃ |
| Example 25a | Synthetic Example 25 | MMAO | Cr (acac) ₃ |
| Example 26a | Synthetic Example 26 | MMAO | Cr(acac)₃ |
| Example 27a | Synthetic Example 27 | MMAO | Cr(acac)₃ |
| Comparative Example 1a | Comparative Synthetic Example 1 | MMAO | Cr(acac)₃ |
| Comparative Example 2a | Comparative Synthetic Example 2 | MMAO | Cr(acac)₃ |
| Comparative Example 3a | Comparative Synthetic Example 3 | MMAO | Cr(acac)₃ |
| Comparative Example 7a | Comparative Synthetic Example 7 | MMAO | Cr(acac)₃ |

### Example 1b

Under an argon gas atmosphere, chromium(III) chloride tetrahydrofuran (Cr(THF)₃Cl₃, 0.05 mmol), the ligand compound (0.5 mmol) according to Synthetic Example 1, and N,N-dimethylanilinium tetrakis(pentafluorophenyl)borate (AB, 0.5 mmol) were put in a flask, dichloromethane (30 mL) was added and stirred for 1 hour, the solvent was removed in vacuum, and the resultant product was dissolved in methylcyclohexane to prepare a catalyst solution of 5 mM (based on Cr).

A Parr reactor with a volume of 600 mL was prepared, vacuum was applied at 120°C for 2 hours, the inside was replaced with argon, and the temperature was reduced to 80°C. Then, methylcyclohexane (180 mL) and TIBAL (300 µmol) were injected, and the catalyst solution (2 mL, 1.0 µmol Cr) was injected thereto. The valve of an ethylene line adjusted to 30 bar was opened to fill the inside of the reactor with ethylene, and then, stirring was performed at 500 rpm for 15 minutes. The ethylene line valve was closed, the reactor was cooled to 0°C using a dry ice/acetone bath, unreacted ethylene was slowly ventilated, and 0.5 mL of nonane (GC internal standard) was injected thereto. After stirring for 10 seconds, 2 mL of a liquid part of the reactor was taken and quenched with water, and an organic part thus obtained was filtered using a PTFE syringe filter to manufacture a GC-FID sample. Then, the distribution of a liquid product was analyzed by GC. To a remaining solution, 400 mL of ethanol/HCl (10 vol% of aqueous 12 M HCl solution) was added, stirring and filtering were performed, and the amount of a solid product was analyzed. The polymer thus obtained was dried in a vacuum oven of 65°C overnight.

### Examples 2b to 27b, and Comparative Examples 1b to 8b

The same method as in Example 1b was performed except for changing the type of the catalyst as in Table 2 below.

**[Table 2]**

| | Catalyst | Cocatalyst | Chromium source |
|---|---|---|---|
| Example 1b | Synthetic Example 1 | AB | Cr(THF)₃Cl₃ |
| Example 2b | Synthetic Example 2 | AB | Cr(THF)₃Cl₃ |
| Example 3b | Synthetic Example 3 | AB | Cr(THF)₃Cl₃ |
| Example 4b | Synthetic Example 4 | AB | Cr(THF)₃Cl₃ |
| Example 5b | Synthetic Example 5 | AB | Cr(THF)₃Cl₃ |
| Example 6b | Synthetic Example 6 | AB | Cr(THF)₃Cl₃ |
| Example 7b | Synthetic Example 7 | AB | Cr(THF)₃Cl₃ |
| Example 8b | Synthetic Example 8 | AB | Cr(THF)₃Cl₃ |
| Example 9b | Synthetic Example 9 | AB | Cr(THF)₃Cl₃ |
| Example 10b | Synthetic Example 10 | AB | Cr(THF)₃Cl₃ |
| Example 11b | Synthetic Example 11 | AB | Cr(THF)₃Cl₃ |
| Example 12b | Synthetic Example 12 | AB | Cr(THF)₃Cl₃ |
| Example 13b | Synthetic Example 13 | AB | Cr(THF)₃Cl₃ |
| Example 14b | Synthetic Example 14 | AB | Cr(THF)₃Cl₃ |
| Example 15b | Synthetic Example 15 | AB | Cr(THF)₃Cl₃ |
| Example 16b | Synthetic Example 16 | AB | Cr(THF)₃Cl₃ |
| Example 17b | Synthetic Example 17 | AB | Cr(THF)₃Cl₃ |
| Example 18b | Synthetic Example 18 | AB | Cr(THF)₃Cl₃ |
| Example 19b | Synthetic Example 19 | AB | Cr(THF)₃Cl₃ |
| Example 20b | Synthetic Example 20 | AB | Cr(THF)₃Cl₃ |
| Example 21b | Synthetic Example 21 | AB | Cr(THF)₃Cl₃ |
| Example 22b | Synthetic Example 22 | AB | Cr(THF)₃Cl₃ |
| Example 23b | Synthetic Example 23 | AB | Cr(THF)₃Cl₃ |
| Example 24b | Synthetic Example 24 | AB | Cr(THF)₃Cl₃ |
| Example 25b | Synthetic Example 25 | AB | Cr(THF)₃Cl₃ |
| Example 26b | Synthetic Example 26 | AB | Cr(THF)₃Cl₃ |
| Example 27b | Synthetic Example 27 | AB | Cr(THF)₃Cl₃ |
| Comparative Example 1b | Comparative Synthetic Example 1 | AB | Cr(THF)₃Cl₃ |
| Comparative Example 2b | Comparative Synthetic Example 2 | AB | Cr(THF)₃Cl₃ |
| Comparative Example 3b | Comparative Synthetic Example 3 | AB | Cr(THF)₃Cl₃ |
| Comparative Example 4b | Comparative Synthetic Example 4 | AB | Cr(THF)₃Cl₃ |
| Comparative Example 5b | Comparative Synthetic Example 5 | AB | Cr(THF)₃Cl₃ |
| Comparative Example 6b | Comparative Synthetic Example 6 | AB | Cr(THF)₃Cl₃ |
| Comparative Example 7b | Comparative Synthetic Example 7 | AB | Cr(THF)₃Cl₃ |
| Comparative Example 8b | Comparative Synthetic Example 8 | AB | Cr(THF)₃Cl₃ |

### Experimental Example 1

The ethylene oligomerization reaction results according to the Examples and Comparative Examples are summarized in Table 3 and Table 4 below.

### (1) Catalyst activity (ton/mol·Cr/hr)

From the total weight (ton) value of the product obtained by combining the weights (ton) of the liquid product and solid product obtained, the catalyst activity was calculated.

### (2) 1-C6 or 1-C8 selectivity

From the GC analysis results of the distribution of a liquid product, the 1-hexene (1-C6) and 1-octene (1-C8) contents were calculated, and the wt% of 1-hexene or 1-octene based on the total weight of the product was calculated.

### (3) Solid (wt%)

The wt% of the solid product based on the total weight of the product was calculated. The solid means insoluble solid which is not dissolved in a solvent, and represents the degree of production of polyethylene with a carbon number of about 40 or more.

**[Table 3]**

| | Catalyst activity (ton/mol·Cr/hr) | 1-C6 or 1-C8 selectivity | | | Solid (wt%) |
|---|---|---|---|---|---|
| | | 1-C6 (wt%) | 1-C8 (wt%) | 1-C6 + 1-C8 (wt%) | |
| Example 1a | 370 | 35.1 | 56.0 | 91.1 | 0.10 |
| Example 2a | 323 | 38.4 | 53.2 | 91.6 | 0.20 |
| Example 3a | 354 | 37.3 | 52.1 | 89.4 | 0.40 |
| Example 4a | 368 | 37.5 | 53.1 | 90.6 | 0.20 |
| Example 5a | 372 | 34.8 | 56.2 | 91.0 | 0.20 |
| Example 6a | 373 | 34.6 | 56.3 | 90.9 | 0.10 |
| Example 7a | 355 | 34.8 | 55.0 | 89.8 | 0.30 |
| Example 8a | 383 | 34.5 | 57.1 | 91.6 | 0.10 |
| Example 9a | 372 | 34.7 | 56.3 | 91.0 | 0.20 |
| Example 10a | 384 | 18.2 | 71.6 | 89.8 | 0.17 |
| Example 25a | 347 | 36.6 | 53.0 | 89.6 | 0.40 |
| Example 26a | 354 | 35.8 | 54.2 | 90.0 | 0.20 |
| Example 27a | 336 | 37.3 | 52.5 | 89.8 | 0.40 |
| Comparative Example 1a | 210 | 40.1 | 48.2 | 88.7 | 0.50 |
| Comparative Example 2a | 151 | 40.3 | 47.9 | 88.2 | 0.70 |
| Comparative Example 3a | 263 | 36.7 | 43.6 | 80.3 | 1.50 |
| Comparative Example 7a | 162 | 41.3 | 47.5 | 88.8 | 0.70 |

**[Table 4]**

| | Catalyst activity (ton/mol·Cr/hr) | 1-C6 or 1-C8 selectivity | | | Solid (wt%) |
|---|---|---|---|---|---|
| | | 1-C6 (wt%) | 1-C8 (wt%) | 1-C6 + 1-C8 (wt%) | |
| Example 1b | 220 | 35.0 | 54.2 | 89.2 | 0.39 |
| Example 2b | 182 | 37.5 | 52.5 | 88.0 | 0.50 |
| Example 3b | 198 | 37.2 | 51.8 | 89.0 | 0.71 |
| Example 4b | 222 | 37.3 | 52.7 | 90.0 | 0.40 |
| Example 5b | 224 | 34.8 | 54.3 | 89.1 | 0.43 |
| Example 6b | 219 | 34.7 | 54.6 | 89.3 | 0.52 |
| Example 7b | 140 | 34.9 | 54.8 | 89.7 | 0.70 |
| Example 8b | 231 | 34.9 | 54.4 | 89.3 | 0.50 |
| Example 9b | 217 | 34.5 | 53.9 | 88.4 | 0.49 |
| Example 10b | 244 | 26.0 | 62.9 | 88.9 | 0.13 |
| Example 11b | 158 | 48.1 | 43.3 | 91.4 | 0.71 |
| Example 12b | 156 | 26.3 | 63.3 | 89.6 | 0.10 |
| Example 13b | 169 | 26.1 | 63.0 | 89.1 | 0.38 |
| Example 14b | 318 | 29.7 | 60.1 | 89.7 | 0.11 |
| Example 15b | 244 | 28.5 | 60.1 | 89.0 | 0.46 |
| Example 16b | 253 | 28.2 | 62.2 | 90.4 | 0.13 |
| Example 17b | 176 | 30.1 | 59.4 | 89.5 | 0.27 |
| Example 18b | 208 | 28.2 | 60.1 | 89.2 | 0.26 |
| Example 19b | 169 | 27.1 | 61.4 | 88.5 | 1.05 |
| Example 20b | 171 | 25.6 | 62.1 | 87.8 | 0.58 |
| Example 21b | 25 | 47.8 | 42.9 | 90.7 | 1. 64 |
| Example 22b | 37 | 43.5 | 46.1 | 89.6 | 0.77 |
| Example 23b | 52 | 48.1 | 42.2 | 90.3 | 0.31 |
| Example 24b | 77 | 43.2 | 46.3 | 88.5 | 0.56 |
| Example 25b | 194 | 37.0 | 51.3 | 88.3 | 0.60 |
| Example 26b | 203 | 36.3 | 52.9 | 89.2 | 0.70 |
| Example 27b | 188 | 38.0 | 51.3 | 89.3 | 1.10 |
| Comparative Example 1b | 0 | - | - | - | - |
| Comparative Example 2b | 0 | - | - | - | - |
| Comparative Example 3b | 43 | 36.6 | 42.0 | 78.6 | 2.40 |
| Comparative Example 4b | 139 | 26.3 | 60.3 | 86.6 | 0.73 |
| Comparative Example 5b | 62 | 26.2 | 61.1 | 87.3 | 1.58 |
| Comparative | 58 | 25.8 | 60.3 | 86.1 | 1.10 |
| Example 6b | | | | | |
| Comparative Example 7b | 0 | - | - | - | - |
| Comparative Example 8b | 116 | 26.4 | 59.9 | 86.3 | 1.36 |

As confirmed in Table 1 and Table 2, the catalyst system including an organochromium compound of the present invention has excellent catalyst activity and shows high selectivity to 1-hexene and 1-octene during polymerizing an olefin-based monomer, thereby enabling more efficient preparation of an alpha-olefin. As shown in Table 3, the catalyst activity was excellent in all of the Examples, and the amount of by-products such as a solid alpha-olefin was reduced when compared to Comparative Examples 1a and 2a, which used ligand compounds in which a methyl substituent having a small size was bonded to phenyl, and Comparative Example 3a which used a ligand compound in which not cycloalkyl but alkyl was bonded to nitrogen. It was found that the selectivity to an alpha-olefin (1-hexene and 1-octene) was even higher. In addition, in the case of using a compound (Comparative Synthetic Example 7) in which bulky substituents were bonded to both meta and para of phenyl, it could be found that the catalyst activity was rather reduced, because the approach of a monomer was difficult due to the excessively bulky catalyst structure like in Comparative Example 7a.

In the results of Table 4, using different types of cocatalysts and chromium sources, the Examples showed improved catalyst activity and alpha-olefin selectivity, and reduced production amount of a solid type alpha-olefin by-products when compared to Comparative Examples 1b and 2b, in which ligand compounds in which a methyl substituent was bonded to a phenyl group were used, and Comparative Examples 3b to 6b, in which ligand compounds in which alkyl was bonded to nitrogen were used.

As described above, in the catalyst of the present invention, cycloalkyl or aryl-fused cycloalkyl is bonded to N, and a bulky substituent is positioned at a para position of phenyl bonded to P, and it could be found that catalyst activity is high, and the production amounts of by-products could be reduced in ethylene oligomerization reaction, and an alpha-olefin could be prepared with high selectivity. In addition, it was confirmed that such effects are intrinsic effects shown by the structure of a ligand compound irrespective of the type of the cocatalyst or chromium source.

## Claims

1. A ligand compound represented by the following Formula 1: 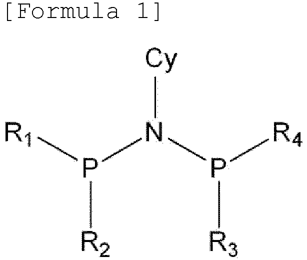 in Formula 1,
Cy is cycloalkyl of 5 to 10 carbon atoms, or cycloalkyl of 5 to 10 carbon atoms, fused with aryl of 6 to 10 carbon atoms, and
R₁ to R₄ are each independently aryl of 6 to 20 carbon atoms, which is para-substituted with a substituent selected from the group consisting of cycloalkyl of 5 to 20 carbon atoms, alkyl of 6 to 20 carbon atoms, alkoxyalkyl of 6 to 20 carbon atoms, arylalkoxyalkyl of 10 to 30 carbon atoms and trialkylsilyl of 3 to 30 carbon atoms,
wherein the cyloalkyl is a nonaromatic cyclic hydrocarbon radical composed of carbon atoms,
wherein the alkyl is a hydrocarbon residue of a linear chain, a cyclic or a branched type,
wherein the alkoxyalkyl is a substituent in which one or more hydrogen of alkyl are substituted with alkoxy,
wherein the arylalkoxyalkyl is a substituent in which one or more hydrogen of alkyl are substituted with arylakoxy,
wherein the trialkylsilyl is a substituent represented by -SiR₃ where each R is independently alkyl, and
wherein the aryl is an arbitrarily substituted benzene ring, or refers to a ring system which may be formed by fusing one or more arbitrary substituents, unless otherwise referred to.

2. The ligand compound according to claim 1, wherein the Cy is any one selected from the group consisting of cyclopentyl, cyclohexyl, cycloheptyl or cyclooctyl, unsubstituted or substituted with alkyl in accordance with the definition provided in the specification of 1 to 6 carbon atoms; dihydroindenyl, tetrahydronaphthyl, 6,7,8,9-tetrahydrobenzo[7]annulenyl, 5,6,7,8,9,10-hexahydrobenzo[8]annulenyl and 9H-fluorenyl, unsubstituted or substituted with alkyl in accordance with the definition provided in the specification of 1 to 6 carbon atoms.

3. The ligand compound according to claim 1, wherein the Cy is any one selected from the following group:
a) 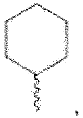
b) 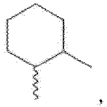
c) 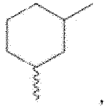
d) 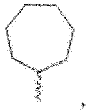
e) 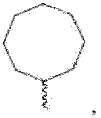
f) 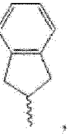
g) 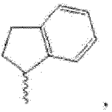
h) 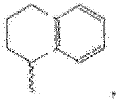
i) 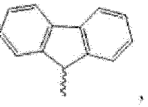
j)

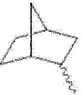

4. The ligand compound according to claim 1, wherein R₁ to R₄ are each independently phenyl which is para-substituted with a substituent selected from the group consisting of cycloalkyl of 6 to 10 carbon atoms, alkyl in accordance with the definition provided in the specification of 8 to 15 carbon atoms, alkoxyalkyl of 9 to 15 carbon atoms, arylalkoxyalkyl of 14 to 20 carbon atoms and trialkylsilyl of 3 to 15 carbon atoms.

5. The ligand compound according to claim 1, wherein R₁ to R₄ are each independently selected from the following group:

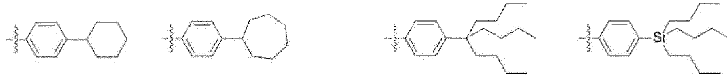

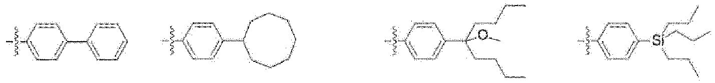

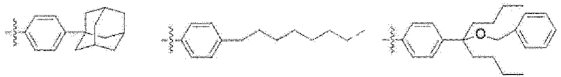

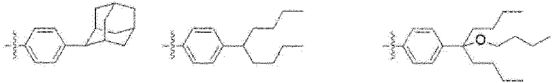

6. An organochromium compound comprising a ligand compound according to any one of the claims 1 to 5 and chromium (Cr) coordinated with the ligand compound.

7. The organochromium compound according to claim 6, wherein one or more unshared electron pairs among N and two P in the ligand compound represented by Formula 1 are coordinated with chromium.

8. A catalyst system comprising chromium, the ligand compound according to any one of claims 1 to 5, and a cocatalyst.

9. The catalyst system according to claim 8, wherein the chromium is derived from a chromium source, and the chromium source comprises one or more selected from the group consisting of chromium(III) acetylacetonate, chromium(III) chloride tetrahydrofuran, chromium(III) 2-ethylhexanoate, chromium(III) acetate, chromium(III) butyrate, chromium(III) pentanoate, chromium(III) laurate, chromium(III) tris(2,2,6,6-tetramethyl-3,5-heptanedionate) and chromium(III) stearate.

10. The catalyst system according to claim 8, wherein the cocatalyst is one or more selected from the group consisting of the following Formulae 2 to 4:
[Formula 2] - [Al(Rₐ)-O]ₐ-
in Formula 2,
each Rₐ is independently a halogen radical, a hydrocarbyl radical of 1 to 20 carbon atoms, or a halogen-substituted hydrocarbyl radical of 1 to 20 carbon atoms, and
a is an integer of 2 or more,
[Formula 3] D(R_{b})₃
in Formula 3,
D is aluminum or boron, and
each R_{b} is independently hydrogen, a halogen radical, a hydrocarbyl radical of 1 to 20 carbon atoms, or a halogen-substituted hydrocarbyl radical of 1 to 20 carbon atoms,
[Formula 4] [L-H]⁺[Z(A)₄]⁻ or [L]⁺[Z(A)₄]⁻
in Formula 4,
L is a neutral or cationic Lewis acid,
Z is an element in group 13, and
each A is independently aryl in accordance with the definition provided in the specification of 6 to 20 carbon atoms or alkyl in accordance with the definition provided in the specification of 1 to 20 carbon atoms, where one or more hydrogen atoms may be substituted with substituents, and the substituent is halogen, hydrocarbyl of 1 to 20 carbon atoms, alkoxy of 1 to 20 carbon atoms, or aryloxy of 6 to 20 carbon atoms,
wherein the alkyl is a hydrocarbon residue of a linear chain, a cyclic or a branched type,
wherein the aryl is an arbitrarily substituted benzene ring, or refers to a ring system which may be formed by fusing one or more arbitrary substituents, unless otherwise referred to.

11. A method of preparing a linear alpha-olefin, the method comprising: oligomerizing ethylene in the presence of the catalyst system of claim 8.

12. The method of preparing a linear alpha-olefin according to claim 11, wherein the linear alpha-olefin is 1-hexene, 1-octene or a mixture of them.

## Patentansprüche

1. Ligandenverbindung, dargestellt durch die folgende Formel 1: 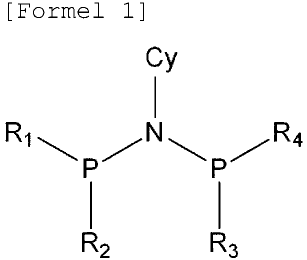 in Formel 1
ist Cy Cycloalkyl mit 5 bis 10 Kohlenstoffatomen oder Cycloalkyl mit 5 bis 10 Kohlenstoffatomen, kondensiert mit Aryl mit 6 bis 10 Kohlenstoffatomen, und
sind R₁ bis R₄ jeweils unabhängig Aryl mit 6 bis 20 Kohlenstoffatomen, das para-substituiert ist mit einem Substituenten, ausgewählt aus der Gruppe, bestehend aus Cycloalkyl mit 5 bis 20 Kohlenstoffatomen, Alkyl mit 6 bis 20 Kohlenstoffatomen, Alkoxyalkyl mit 6 bis 20 Kohlenstoffatomen, Arylalkoxyalkyl mit 10 bis 30 Kohlenstoffatomen und Trialkylsilyl mit 3 bis 30 Kohlenstoffatomen,
wobei das Cycloalkyl ein nichtaromatischer cyclischer Kohlenwasserstoffrest ist, zusammengesetzt aus Kohlenstoffatomen,
wobei das Alkyl ein Kohlenwasserstoffrest einer linearen Kette, eines cyclischen oder eines verzweigten Typs ist,
wobei das Alkoxyalkyl ein Substituent ist, in dem ein oder mehrere Wasserstoffe von Alkyl mit Alkoxy substituiert sind,
wobei das Arylalkoxyalkyl ein Substituent ist, in dem ein oder mehrere Wasserstoffe von Alkyl mit Aryloxy substituiert sind,
wobei das Trialkylsilyl ein Substituent ist, dargestellt durch -SiR₃ , wobei jedes R unabhängig Alkyl ist, und
wobei das Aryl ein beliebig substituierter Benzolring ist oder sich auf ein Ringsystem bezieht, das durch Kondensieren eines oder mehrerer beliebiger Substituenten gebildet werden kann, sofern nicht anders angegeben.

2. Ligandenverbindung nach Anspruch 1, wobei das Cy eines ist, ausgewählt aus der Gruppe, bestehend aus Cyclopentyl, Cyclohexyl, Cycloheptyl oder Cyclooctyl, unsubstituiert oder substituiert mit Alkyl gemäß der in der Beschreibung bereitgestellten Definition von 1 bis 6 Kohlenstoffatomen; Dihydroindenyl, Tetrahydronaphthyl, 6,7,8,9-Tetrahydrobenzo[7]annulenyl, 5,6,7,8,9,10-Hexahydrobenzo[8]annulenyl und 9H-Fluorenyl, unsubstituiert oder substituiert mit Alkyl gemäß der in der Beschreibung bereitgestellten Definition von 1 bis 6 Kohlenstoffatomen.

3. Ligandenverbindung nach Anspruch 1, wobei das Cy eines ist, ausgewählt aus der folgenden Gruppe:
a) 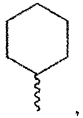
b) 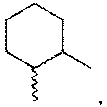
c) 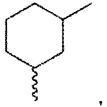
d) 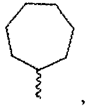
e) 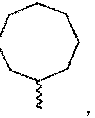
f) 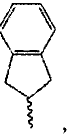
g) 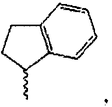
h) 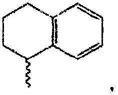
i) 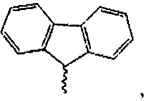
j)

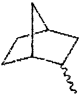

4. Ligandenverbindung nach Anspruch 1, wobei R₁ bis R₄ jeweils unabhängig Phenyl sind, das para-substituiert ist mit einem Substituenten, ausgewählt aus der Gruppe, bestehend aus Cycloalkyl mit 6 bis 10 Kohlenstoffatomen, Alkyl gemäß der in der Beschreibung bereitgestellten Definition von 8 bis 15 Kohlenstoffatomen, Alkoxyalkyl mit 9 bis 15 Kohlenstoffatomen, Arylalkoxyalkyl mit 14 bis 20 Kohlenstoffatomen und Trialkylsilyl mit 3 bis 15 Kohlenstoffatomen.

5. Ligandenverbindung nach Anspruch 1, wobei R₁ bis R₄ jeweils unabhängig ausgewählt sind aus der folgenden Gruppe:

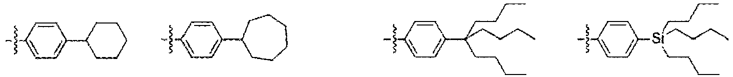

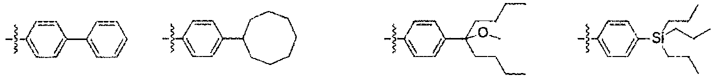

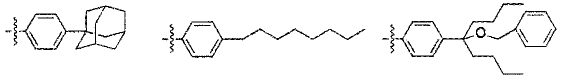

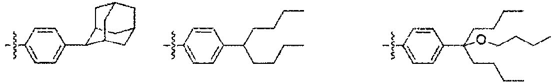

6. Organochromverbindung, umfassend eine Ligandenverbindung nach einem der Ansprüche 1 bis 5 und Chrom (Cr), koordiniert mit der Ligandenverbindung.

7. Organochromverbindung nach Anspruch 6, wobei ein oder mehrere ungeteilte Elektronenpaare unter N und zwei P in der Ligandenverbindung, dargestellt durch Formel 1, mit Chrom koordiniert sind.

8. Katalysatorsystem, umfassend Chrom, die Ligandenverbindung nach einem der Ansprüche 1 bis 5 und einen Cokatalysator.

9. Katalysatorsystem nach Anspruch 8, wobei das Chrom von einer Chromquelle abgeleitet ist und die Chromquelle eines oder mehrere umfasst, ausgewählt aus der Gruppe, bestehend aus Chrom(III)acetylacetonat, Chrom(III)chloridtetrahydrofuran, Chrom(III)-2-ethylhexanoat, Chrom(III)acetat, Chrom(III)butyrat, Chrom(III)pentanoat, Chrom(III)laurat, Chrom(III)tris(2,2,6,6-tetramethyl-3,5-heptandionat) und Chrom(III)stearat.

10. Katalysatorsystem nach Anspruch 8, wobei der Cokatalysator einer oder mehrere ist, ausgewählt aus der Gruppe, bestehend aus den folgenden Formeln 2 bis 4:
[Formel 2] - [Al(Rₐ)-O]ₐ-
in Formel 2,
ist jedes Rₐ unabhängig ein Halogenrest, ein Kohlenwasserstoffrest mit 1 bis 20 Kohlenstoffatomen oder ein halogensubstituierter Kohlenwasserstoffrest mit 1 bis 20 Kohlenstoffatomen, und
a ist eine ganze Zahl von 2 oder mehr,
[Formel 3] D(R_{b})₃
in Formel 3,
ist D Aluminium oder Bor, und
ist jedes R_{b} unabhängig Wasserstoff, ein Halogenrest, ein Kohlenwasserstoffrest mit 1 bis 20 Kohlenstoffatomen oder ein halogensubstituierter Kohlenwasserstoffrest mit 1 bis 20 Kohlenstoffatomen,
[Formel 4] [L-H]⁺[Z(A)₄]⁻ oder [L]⁺[Z(A)₄]⁻
in Formel 4,
ist L eine neutrale oder kationische Lewis-Säure,
ist Z ein Element in Gruppe 13, und
ist jedes A unabhängig Aryl gemäß der in der Beschreibung bereitgestellten Definition von 6 bis 20 Kohlenstoffatomen oder Alkyl gemäß der in der Beschreibung bereitgestellten Definition von 1 bis 20 Kohlenstoffatomen, wobei ein oder mehrere Wasserstoffatome mit Substituenten substituiert sein können, und ist der Substituent Halogen, Kohlenwasserstoff mit 1 bis 20 Kohlenstoffatomen, Alkoxy mit 1 bis 20 Kohlenstoffatomen oder Aryloxy mit 6 bis 20 Kohlenstoffatomen,
wobei das Alkyl ein Kohlenwasserstoffrest einer linearen Kette, eines cyclischen oder eines verzweigten Typs ist,
wobei das Aryl ein beliebig substituierter Benzolring ist oder sich auf ein Ringsystem bezieht, das durch Kondensieren eines oder mehrerer beliebiger Substituenten gebildet werden kann, sofern nicht anders angegeben.

11. Verfahren zur Herstellung eines linearen alpha-Olefins, wobei das Verfahren umfasst: Oligomerisieren von Ethylen in Gegenwart des Katalysatorsystems nach Anspruch 8.

12. Verfahren zur Herstellung eines linearen alpha-Olefins nach Anspruch 11, wobei das lineare alpha-Olefin 1-Hexen, 1-Octen oder eine Mischung davon ist.

## Revendications

1. Composé de ligand représenté par la formule 1 ci-après : 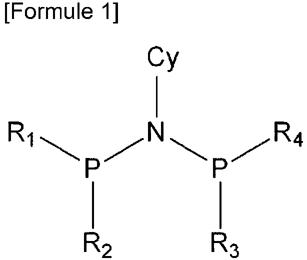 dans la Formule 1,
Cy est un cycloalkyle de 5 à 10 atomes de carbone ou un cycloalkyle de 5 à 10 atomes de carbone, fusionné avec un aryle de 6 à 10 atomes de carbone, et
R₁ à R₄ sont chacun indépendamment un aryle de 6 à 20 atomes de carbone, qui est para-substitué par un substituant sélectionné dans le groupe constitué d'un cycloalkyle de 5 à 20 atomes de carbone, un alkyle de 6 à 20 atomes de carbone, un alkoxyalkyle de 6 à 20 atomes de carbone, un arylalkoxyalkyle de 10 à 30 atomes de carbone et un trialkylsilyle de 3 à 30 atomes de carbone,
dans lequel le cyloalkyle est un radical d'hydrocarbure cyclique non aromatique composé d'atomes de carbone,
dans lequel l'alkyle est un résidu d'hydrocarbure d'une chaîne linéaire, d'un type cyclique ou ramifié,
dans lequel l'alkoxyalkyle est un substituant dans lequel un ou plusieurs atomes d'hydrogène de l'alkyle sont substitués par un alkoxy,
dans lequel l'arylalkoxyalkyle est un substituant dans lequel un ou plusieurs atomes d'hydrogène de l'alkyle sont substitués par un arylalkoxy,
dans lequel le trialkylsilyle est un substituant représenté par -SiR₃ où chaque R est indépendamment un alkyle, et
dans lequel l'aryle est un cycle de benzène substitué arbitrairement, ou fait référence à un système de cycle qui peut être formé en fusionnant un ou plusieurs substituants arbitraires, sauf référence contraire.

2. Composé de ligand selon la revendication 1, dans lequel le Cy est un élément sélectionné dans le groupe constitué d'un cyclopentyle, cyclohexyle, cycloheptyle ou cyclooctyle, non substitué ou substitué par un alkyle selon la définition fournie dans la spécification de 1 à 6 atomes de carbone ; un dihydroindényle, un tétrahydronaphtyle, un 6,7,8,9-tétrahydrobenzo[7]annulenyle, un 5,6,7,8,9,10-hexahydrobenzo[8]annulenyle et un 9H-fluorenyle, non substitué ou substitué par un alkyle selon la définition fournie dans la spécification de 1 à 6 atomes de carbone.

3. Composé de ligand selon la revendication 1, dans lequel le Cy est un élément sélectionné dans le groupe suivant :
a) 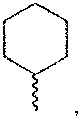
b) 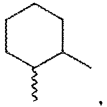
c) 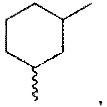
d) 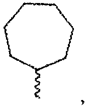
e) 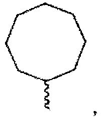
f) 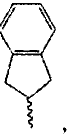
g) 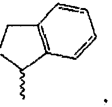
h) 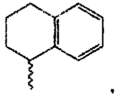
i) 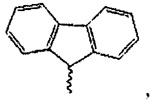
j)

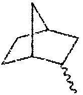

4. Composé de ligand selon la revendication 1, dans lequel R₁ à R₄ sont chacun indépendamment un phényle qui est para-substitué par un substituant sélectionné dans le groupe constitué d'un cycloalkyle de 6 à 10 atomes de carbone, un alkyle selon la définition fournie dans la spécification de 8 à 15 atomes de carbone, un alkoxyalkyle de 9 à 15 atomes de carbone, un arylalkoxyalkyle de 14 à 20 atomes de carbone et un trialkylsilyle de 3 à 15 atomes de carbone.

5. Composé de ligand selon la revendication 1, dans lequel R₁ à R₄ sont chacun indépendamment sélectionnés dans le groupe suivant :

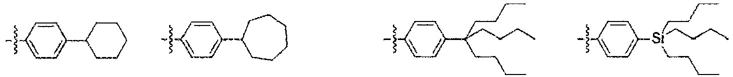

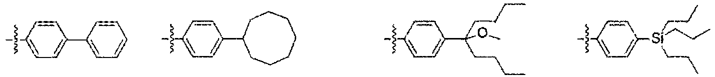

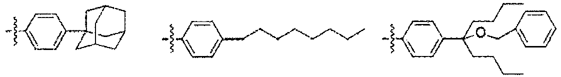

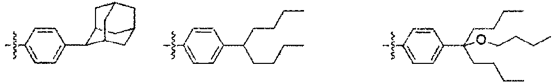

6. Composé de chrome organique comprenant un composé de ligand selon l'une quelconque des revendications 1 à 5 et un chrome (Cr) coordonné avec le composé de ligand.

7. Composé de chrome organique selon la revendication 6, dans lequel une ou plusieurs paires d'électrons non partagés parmi N et deux P dans le composé de ligand représenté par la formule 1 sont coordonnées avec le chrome.

8. Système de catalyseur comprenant un chrome, le composé de ligand selon l'une quelconque des revendications 1 à 5, et un cocatalyseur.

9. Système de catalyseur selon la revendication 8, dans lequel le chrome est dérivé d'une source de chrome, et la source de chrome comprend un ou plusieurs éléments sélectionnés dans le groupe constitué d'un acétylacétonate de chrome(III), chlorure de tétrahydrofurane de chrome(III), 2-éthylhexanoate de chrome(III), acétate de chrome(III), butyrate de chrome(III), pentanoate de chrome(III), laurate de chrome(III), tris(2,2,6,6-tétraméthyl-3,5-heptanedionate) de chrome(III) et stéarate de chrome(III).

10. Système de catalyseur selon la revendication 8, dans lequel le cocatalyseur est un ou plusieurs éléments sélectionnés dans le groupe constitué des Formules 2 à 4 ci-après :
[Formule 2] -[Al(Rₐ)-O]ₐ-
dans la Formule 2,
chaque Rₐ est indépendamment un radical halogène, un radical hydrocarbyle de 1 à 20 atomes de carbone ou un radical hydrocarbyle de 1 à 20 atomes de carbone substitué par un halogène, et
a est un nombre entier de 2 ou plus,
[Formule 3] D(R_{b})₃
dans la Formule 3,
D est un aluminium ou un bore, et
chaque R_{b} est indépendamment un hydrogène, un radical halogène, un radical hydrocarbyle de 1 à 20 atomes de carbone ou un radical hydrocarbyle de 1 à 20 atomes de carbone substitué par un halogène,
[Formule 4] [L-H]⁺[Z(A)₄]⁻ ou [L]⁺[Z(A)₄]⁻
dans la Formule 4,
L est un acide de Lewis neutre ou cationique,
Z est un élément du Groupe 13, et
chaque A est indépendamment un aryle selon la définition fournie dans la spécification de 6 à 20 atomes de carbone ou un alkyle selon la définition fournie dans la spécification de 1 à 20 atomes de carbone, où un ou plusieurs atomes d'hydrogène peuvent être substitués par des substituants, et le substituant est un halogène, un hydrocarbyle de 1 à 20 atomes de carbone, un alkoxy de 1 à 20 atomes de carbone ou un aryloxy de 6 à 20 atomes de carbone,
dans lequel l'alkyle est un résidu d'hydrocarbure d'une chaîne linéaire, d'un type cyclique ou ramifié,
dans lequel l'aryle est un cycle de benzène substitué arbitrairement, ou fait référence à un système de cycle qui peut être formé en fusionnant un ou plusieurs substituants arbitraires, sauf référence contraire.

11. Procédé de préparation d'une alpha-oléfine linéaire, le procédé consistant à : oligomériser un éthylène en présence du système de catalyseur selon la revendication 8.

12. Procédé de préparation d'une alpha-oléfine linéaire selon la revendication 11, dans lequel l'alpha-oléfine linéaire est 1-hexène, 1-octène ou un mélange de ces derniers.
